# EUROPEAN PATENT APPLICATION

(11) **EP 3 524 254 A1**
(43) Date of publication of application: **14.08.2019**
(21) Application number: 17850983.2
(22) Date of filing: 14.09.2017
(51) Int. Cl.: A61K 36/064, A23L 31/00, A23L 31/15, A23L 33/125, A61K 8/60, A61K 31/715, A61K 35/744, A61K 36/02, A61P 1/16, A61P 1/18, A61P 3/10, A61P 9/00, A61P 9/04, A61P 9/06, A61P 9/10, A61P 11/00, A61P 13/12, A61P 17/00, A61P 17/02, A61P 19/00, A61P 19/10

(54) **Lin28a ACTIVATOR AND USE THEREFOR**

(30) Priority: 14.09.2016 JP 2016179598
(71) Applicant: Tes Holdings Co., Ltd., Tokyo 110-0015 (JP)
(72) Inventor: HAYASHI Akio, Tokyo 113-0033 (JP)
(74) Representative: De Clercq & Partners
(86) International application number: PCT/JP2017/033257
(87) International publication number: WO 2018/052082

(57) **Abstract**

A Lin28a activator includes a polysaccharide containing one or more selected from the group consisting of ribose, xylose, and derivatives thereof as an active ingredient. A dedifferentiation inducer of a monocyte includes a macrophage colony-stimulating factor and a polysaccharide containing one or more selected from the group consisting of ribose, xylose, and derivatives thereof as active ingredients. A therapeutic agent against diseases relating to damage in cells, tissues, or organs includes a polysaccharide containing one or more selected from the group consisting of ribose, xylose, and derivatives thereof as an active ingredient. A food or beverage product for preventing or alleviating diseases relating to damage in cells, tissues, or organs includes a polysaccharide containing one or more selected from the group consisting of ribose, xylose, and derivatives thereof. A cosmetic product includes a polysaccharide containing one or more selected from the group consisting of ribose, xylose, and derivatives thereof.

## Description

### Technical Field

The present invention relates to a Lin28a activator and use therefor. Specifically, the present invention relates to a Lin28a activator, a dedifferentiation inducer of monocytes, a therapeutic agent, a food or beverage product, and a cosmetic product containing a polysaccharide as an active ingredient.

Priority is claimed on Japanese Patent Application No. 2016-179598, filed on September 14, 2016, the content of which is incorporated herein by reference.

### Background Art

Examples of a fundamental treatment method against diseases include a method of recovering functions of cells, tissues, or organs by performing repair using a stem cell in a case where cells, tissues, or organs are damaged, that is, regeneration treatment. In recent years, for regeneration treatment, not only a method of forming targeted tissues or organs from a stem cell and implanting thereof but also a method of injecting a precursor cell of a stem cell or a tissue cell into a disease site and performing regeneration and repair on the disease site, that is, utilization of a cell medicine have been examined.

However, a large amount of cells are required to inject a precursor cell of a stem cell or a tissue cell into a disease site.

Examples of a method of producing a stem cell used in a cell medicine include a method of dedifferentiation to a stem cell by culturing a monocyte derived from peripheral blood in the presence of a specific cytokine such as macrophage colony-stimulating factor (M-CSF) and the like (for example, refer to Non-Patent Documents 1 and 2).

In addition, examples of a method of efficiently producing a stem cell include a method of culturing a monocyte derived from peripheral blood in the presence of M-CSF and at least one selected from the group consisting of ganglioside (glycolipid), and water-soluble plant extract and dedifferentiating thereof and the like (for example, refer to Patent Document 1).

On the other hand, Lin28a is an RNA binding protein transiently expressed in an embryonic period and is recognized to be expressed in ES cells as well, but it is known that expression is lowered in accordance with the progress of the dedifferentiation. It was published by Yamanaka in 2007 that iPS cells can be established by introducing four genes (Oct3/4, SOX2, C-myc, Klf4) to human cells (for example, refer to Non-Patent Document 3). In addition, it was also shown by James Thomson on the same date that a cell having ES cell-like pluripotency can be established by introducing four genes (Oct3/4, SOX2, Nanog, Lin28a) to human cells (for example, refer to Non-Patent Document 4). From these, it is determined that Lin28a is necessary for maintaining or inducing an undifferentiated state.

The establishment of iPS cells due to Yamanaka's four factors could be considered difficult in somatic cells derived from old people. However, it was published that iPS cells are also induced from somatic cells derived from an old person who is 101 years old by introducing Nanog and Lin28a, in addition to Yamanaka's four factors (for example, refer to Non-Patent Document 5). With this, great attention was paid to potentials of Lin28a contributing to initialization of senescent cells. In addition, in 2013, there published an interesting article on an impact in a case where expression of Lin28a is maintained not only in the embryonic period but also in an adult body (for example, refer to Non-Patent Document 6). With this, Lin28a came to be definitively acknowledged as a tissue repair factor. That is, it was reported that, by maintaining expression of Lin28a even in an adult body, hair growth is promoted, in an ear tissue, a hole opened for individual identification is repaired and closed, and in a case of a baby mouse, a fingertip cut for individual identification is regenerated.

### Citation List

### Patent Literature

[Patent Document 1] Republished PCT International Publication No. WO2010/061781 of the PCT International Publication for Patent Applications Non-Patent Literature

[Non-Patent Document 1] Ruhnke M, et al., "Differentiation of in vitro-modified human peripheral blood monocytes into hepatocyte-like and pancreatic islet-like cells", Gastroenterology, vol. 128, no. 7, p1774-1786, 2005.
[Non-Patent Document 2] Zhao Y, et al., "A human peripheral blood monocyte-derived subset acts as pluripotent stem cells", PNAS, vol. 100, no. 5, p2426-2431, 2003.
[Non-Patent Document 3] Yamanaka S, et al., "Induction of Pluripotent Stem Cells from Adult Human Fibroblasts by Defined Factors", Cell, vol. 131, issue 5, p861-872, 2007.
[Non-Patent Document 4] Thomson J A, et al., "Induced pluripotent stem cell lines derived from human somatic cells", Science, vol. 318, issue 5858, p1917-1920, 2007.
[Non-Patent Document 5] Lapasset L, et al., "Rejuvenating senescent and centenarian human cells by reprogramming through the pluripotent state", Genes Dev., vol. 25, p2248-2253, 2011.
[Non-Patent Document 6] Ng Shyh-Chang, et al., "Lin28 Enhances Tissue Repair by Reprogramming Cellular Metabolism", Cell, vol. 155, issue 4, p778-792, 2013.

### Summary of Invention

### Technical Problem

In a method of producing stem cells disclosed in Patent Document 1, it is possible to efficiently dedifferentiate a monocyte into a stem cell by containing at least one selected from ganglioside, and water-soluble plant extract, along with M-CSF. However, a substance that can induce further efficient dedifferentiation was sought.

In addition, the reason why Lin28a which is an RNA binding protein behaves like a growth factor, including the total elucidation of the function, has not been determined, and hitherto, it has only been elucidated that Lin28a accelerates the metabolism of cells. In addition, a substance that induces expression of Lin28a and activates Lin28a has not been known.

Therefore, in view of the above circumstance, the present invention provides a new Lin28a activator and an efficient dedifferentiation inducer.

### Solution to Problem

As a result of intensive studies to achieve the above object, the present inventors found that a polysaccharide contained in a culture product of a specific yeast activates Lin28a and efficiently dedifferentiates a monocyte into a stem cell, thereby completing the present invention.

That is, the present invention includes the following aspects.

A Lin28a activator according to a first aspect of the present invention includes a polysaccharide containing one or more selected from the group consisting of ribose, xylose, and derivatives thereof as an active ingredient.

In the Lin28a activator according to the first aspect, the polysaccharide may further include one or more selected from the group consisting of galactose, mannose, glucose, N-acetylglucosamine, rhamnose, N-acetylgalactosamine, and derivatives thereof.

In the Lin28a activator according to the first aspect, a molecular weight of the polysaccharide maybe 3,500 or greater.

In the Lin28a activator according to the first aspect, the polysaccharide may be derived from microorganisms.

In the Lin28a activator according to the first aspect, the microorganism may be yeast, microalgae, Lactobacillus, or Aspergillus.

In the Lin28a activator according to the first aspect, the yeast may be Saccharomyces boulardii.

A dedifferentiation inducer of a monocyte according to a second aspect of the present invention includes a macrophage colony-stimulating factor and a polysaccharide containing one or more selected from the group consisting of ribose, xylose, and derivatives thereof as active ingredients.

In the dedifferentiation inducer of a monocyte according to the second aspect, the polysaccharide may further contain one or more selected from the group consisting of galactose, mannose, glucose, N-acetylglucosamine, rhamnose, N-acetylgalactosamine, and derivatives thereof.

In the dedifferentiation inducer of a monocyte according to the second aspect, a molecular weight of the polysaccharide may be 3,500 or greater.

A therapeutic agent against diseases relating to damage in cells, tissues, or organs according to a third aspect of the present invention includes a polysaccharide containing one or more selected from the group consisting of ribose, xylose, and derivatives thereof as an active ingredient.

In the therapeutic agent against diseases relating to damage in cells, tissues, or organs according to the third aspect, the disease may be external injury, pancreatitis, radiation damage, dermatomyositis, polymyositis, necrotizing fasciitis, chronic bronchitis, fracture, osteoporosis, osteochondral fracture, osteochondritis, dilated cardiomyopathy, myocardial infarction, ischemic cardiomyopathy, heart failure, myocardial hypertrophy, congestive heart failure, restenosis, arrhythmia, atherosclerosis, vasculitis, peripheral neuropathy, neuropathic pain, stroke, encephalitis, meningitis, diabetic neuropathy, attention deficit disorder, autism, Alzheimer's disease, Parkinson's disease, Creutzfeldt-Jakob disease, brain trauma, spinal cord trauma, cerebral ischemia, cirrhosis, chronic hepatitis, chronic renal failure, glomerular nephritis, renal ischemia, diabetes, atopic dermatitis, or graft versus host disease.

A food or beverage product for preventing or alleviating diseases relating to damage in cells, tissues, or organs according to a fourth aspect of the present invention includes a polysaccharide containing one or more selected from the group consisting of ribose, xylose, and derivatives thereof.

In the food or beverage product for preventing or alleviating diseases relating to damage in cells, tissues, or organs according to the fourth aspect, the disease may be external injury, pancreatitis, radiation damage, dermatomyositis, polymyositis, necrotizing fasciitis, chronic bronchitis, fracture, osteoporosis, osteochondral fracture, osteochondritis, dilated cardiomyopathy, myocardial infarction, ischemic cardiomyopathy, heart failure, myocardial hypertrophy, congestive heart failure, restenosis, arrhythmia, atherosclerosis, vasculitis, peripheral neuropathy, neuropathic pain, stroke, encephalitis, meningitis, diabetic neuropathy, attention deficit disorder, autism, Alzheimer's disease, Parkinson's disease, Creutzfeldt-Jakob disease, brain trauma, spinal cord trauma, cerebral ischemia, cirrhosis, chronic hepatitis, chronic renal failure, glomerular nephritis, renal ischemia, diabetes, atopic dermatitis, or graft versus host disease.

A cosmetic product according to a fifth aspect of the present invention includes a polysaccharide containing one or more selected from the group consisting of ribose, xylose, and derivatives thereof.

### Advantageous Effects of Invention

According to the Lin28a activator of the aspects, it is possible to efficiently activate Lin28a. According to the dedifferentiation inducer of the aspects, it is possible to efficiently induce dedifferentiation of differentiated cells.

### Brief Description of Drawings

FIG. 1 is a drawing showing a result of comparison of inducing ability of expression of Lin28a in a mouse vascular endothelial cell line TKD2 cell obtained by adding various substances to a culture medium in Example 1.
FIG. 2 is a drawing showing a result of comparison of inducing ability of expression of Lin28a in a mouse vascular endothelial cell line TKD2 cell obtained by adding culture products using different culture methods of Saccharomyces boulardii to a culture medium in Example 2.
FIG. 3 is a drawing showing an aspect of proliferation of human mononucleosis obtained by adding various substances to a culture medium in Example 3.
FIG. 4 is a drawing showing a result of comparison of inducing ability of expression of Lin28a in a human fibroblast obtained by adding a culture product of each microorganism to a culture medium in Example 4.
FIG. 5 is a drawing showing a result of comparison of inducing ability of expression of Lin28a in a human fibroblast obtained by adding a substance in a dialysis external liquid and exocrine secretion (substance in dialysis internal liquid) of Saccharomyces boulardii to a culture medium in Example 5.
FIG. 6 is a drawing showing a result of comparison of inducing ability of expression of Lin28a in a human fibroblast obtained by adding each elution substance to a culture medium in Example 6.
FIG. 7A is a graph showing a result of monosaccharide composition analysis of exocrine secretion (before purification by anion exchange chromatography) of Saccharomyces boulardii in Example 6.
FIG. 7B is a graph showing a result of monosaccharide composition analysis of a substance (after purification by anion exchange chromatography) eluted at 300 mM of NaCl in anion exchange chromatography using exocrine secretion of Saccharomyces boulardii in Example 6.
FIG. 8 is a drawing showing a result of comparison of inducing ability of expression of Lin28a, Sirt1, and TERT in a human fibroblast in which exocrine secretion of Saccharomyces boulardii is added to the culture medium in Example 7.
FIG. 9 is a graph in which the number of cells of rat bone marrow cell obtained by adding exocrine secretion matter of Saccharomyces boulardii having different concentrations to the culture medium and culturing thereof is compared in Example 8.

### Description of Embodiments

### <<Lin28a Activator>>

In one embodiment, the present invention provides a Lin28a activator including a polysaccharide containing one or more selected from the group consisting of ribose, xylose, and derivatives thereof as an active ingredient.

According to the Lin28a activator of the present embodiment, it is possible to effectively induce expression of Lin28a and activate Lin28a.

### <Lin28a>

In general, Lin28a is an RNA binding protein transiently expressed in an embryonic period, and is acknowledged to be expressed in ES cells as well, but expression is lowered in accordance with the progress of the differentiation. In addition, it is reported that by maintaining expression of Lin28a in an adult body, hair growth is promoted, in an ear tissue, a hole opened for individual identification is repaired and closed, and in a case of a baby mouse, a fingertip cut for individual identification is regenerated. For this reason, Lin28a is recognized as a tissue repair factor. Therefore, it is possible to repair damage in cells, tissues, or organs by inducing expression of Lin28a and activating Lin28a.

The present inventors found a polysaccharide contained in a culture product of Saccharomyces boulardii, as shown in the examples to be described later, as a substance that induces expression of Lin28a and activates Lin28a.

### <Polysaccharide>

The polysaccharide contained in the Lin28a activator of the present embodiment includes one or more selected from the group consisting of ribose, xylose, and derivatives thereof.

In addition, examples of the derivatives of ribose and xylose include amino sugar in which a hydroxy group (-OH) contained in the monosaccharide is substituted with an amino group (-NH₂), an uronic acid in which a terminal hydroxymethyl group (-CH₂OH) of a main chain is substituted with a carboxy group (-COOH), and the like.

Among these, the polysaccharide preferably includes ribose and xylose. As shown in the examples to be described later, the polysaccharide containing ribose and xylose has a prominent Lin28a activating effect.

The ratio (R/X) of a molar amount of ribose with respect to a molar amount of xylose contained in the polysaccharide is preferably equal to or more than 1/1 and equal to or less than 10/1, more preferably equal to or more than 3/1 and equal to or less than 9/1, and further more preferably equal to or more than 5/1 and equal to or less than 8/1.

In addition, the polysaccharide may further include one or more selected from the group consisting of galactose, mannose, glucose, N-acetylglucosamine, rhamnose, N-acetylgalactosamine, and derivatives thereof.

In addition, examples of the derivatives of the above-described six kinds of monosaccharides include the same as exemplified as the above-described derivatives of ribose and xylose.

In a case where the polysaccharide contains one or more selected from the group consisting of galactose, mannose, glucose, N-acetylglucosamine, rhamnose, N-acetylgalactosamine, and derivatives thereof, the ratio (A/B) of a total molar amount (A) of ribose, xylose, and derivatives thereof with respect to a total molar amount (B) of the six monosaccharides and derivatives thereof contained in the polysaccharide can be equal to or more than 1/2 and equal to or less than 100/1, for example, and can be equal to or more than 1/1 and equal to or less than 50/1, for example. As the ratio of the total molar amount (A) of ribose, xylose, and derivatives thereof with respect to the total molar amount (B) of the above-described six kinds of monosaccharides and derivatives thereof contained in the polysaccharide can more effectively activate Lin28a.

A molecular weight of the polysaccharide is, as shown in the examples to be described later, preferably equal to or more than 3,500, and more preferably equal to or more than 3,500 and equal to or less than 15,000. By setting the molecular weight of the polysaccharide to be an upper limit value or more, it is possible to exhibit more effective Lin28a activation.

### [Microorganisms]

The above-described polysaccharide contained in the present embodiment may be a chemically synthesized one, or may be those obtained from microorganisms.

Examples of the microorganisms from which the above-described polysaccharide is derived include yeast, microalgae, Lactobacillus, Aspergillus, and the like, and are not limited thereto. Among these, the microorganism is preferably yeast.

Examples of the yeast include Saccharomyces boulardii and the like.

In general, Saccharomyces boulardii is fruit yeast obtained by a French microbiologist Dr. Boulard, who found that a local person in Vietnam mitigates diarrhea from cholera by drinking a beverage made of lychee, by being isolated from the beverage product.

The Saccharomyces boulardii is not particularly limited, and specific examples thereof include ATCC MYA-796 strain or ATCC MYA-797 strain.

The microalgae are divided into prokaryote and eukaryote. Examples of the prokaryote include blue-green algae, primordial green algae, and the like. Examples of the eukaryote include gold algae, haptophyte, yellow-green algae, true eye algae, diatom, dinoflagellate, raphidophyte, cryptophyte, euglena algae, prasinophyte, and the like. Among these, the microalgae are preferably blue-green algae.

Examples of the blue-green algae include algae belonging to genus Halothece, genus Dactylococcosis, genus Cyanothece, genus Orthospira, genus Spirulina, genus Halo Spirulina, genus Geitlerinema, genus Prochlorococcus, genus Synechococcus, genus Lyngbya, genus Moorea, Trichodesmium, Oscillatoria, and the like.

Examples of the Lactobacillus include genus Lactobacillus, genus Leuconostoc, genus Lactooccus, genus Enterococcus, genus Pediococcus, genus Weissella, and the like.

Examples of the genus Lactobacillus include Lactobacillus plantarum, Lactobacillus casei, Lactobacillus buchneri, Lactobacillus rhamnosus, and the like.

Examples of the genus Leuconostoc include Leuconostoc citreum, Leuconostoc mesenteroides, and the like.

Examples of the genus Lactooccus include Lactococcus lactis, Lactococcus rhamnosus, and the like.

Examples of the genus Enterococcus include Enterococcus facialis, Enterococcus faecium, and the like.

Examples of the genus Pediococcus include Pediococcus acildilactici and the like.

Examples of the genus Weissella include Weissella confuse, Weissella oryze, and the like.

Examples of the genus Aspergillus include Aspergillus oryzae, Aspergillus sojae, Aspergillus tamarii, Aspergillus kawachii, Aspergillus awamori, Aspergillus brasiliensis, Aspergillus saitoi, and the like.

In the Lin28a activator of the present embodiment, a culture product of microorganisms may be used as a composition containing the polysaccharide. Or only the polysaccharide may be separated from the culture product of microorganisms, purified, and used. The culture product of microorganisms includes a crush liquid of microorganisms (for example, yeast extract), a suspension, and a culture supernatant. In addition, the culture product of microorganisms may be liquid, and may be a dry body

(for example, dry bacteria, those obtained by drying a culture supernatant, and the like). In addition, in the Lin28a activator of the present embodiment, a composition in a state of including a bacterial cell of microorganisms may be used as a composition containing the polysaccharide.

Although it varies depending the use, in a case of being used in a medical product, an external cosmetic product, or the like, it is demanded that it has a high effect in a small amount, a high safety and high purity because it is directly administered or injected (for example, oral, transdermal, intravenous, intraperitoneal, and the like) in the body of a subject. In this regard, in a case of being used in a medical product, an external cosmetic product, or the like, those obtained by separating and purifying only the polysaccharide contained in a culture product of microorganisms are preferably used.

On the other hand, in a case of being used in a food or beverage product such as a health food, an oral cosmetic, or culturing for dedifferentiation of monocytes, a bacterial cell of microorganisms contains abundant nutrition such as mineral and amino acids, and the like. In this regard, in the case of being used in a food or beverage product such as a health food, an oral cosmetic, or culturing for dedifferentiation of monocytes, those in a state of including a bacterial cell of microorganisms are preferably used.

In the present embodiment, a structure of the polysaccharide contained in the culture product of microorganisms is not identified, but a molecular weight is preferably approximately equal to or more than 3,500 and equal to or less than 15,000. In addition, a pH in a liquid state of the culture product may be equal to or more than 4 and equal to or less than 9, for example.

The culture product of microorganisms in the present embodiment can be obtained by culturing the microorganisms by using a general culture medium used in culturing of microorganisms.

In a case where the microorganisms are yeast or microalgae, examples of the culture medium include YPD culture medium (containing 2% peptone, 1% yeast extract, 2% glucose), YPD culture medium in which some glucose is substituted with rhamnose, YPD culture medium in which some glucose is substituted with ribose, and the like, and are not limited thereto. Among these, the culture medium is preferably the YPD culture medium in which some glucose is substituted with rhamnose or the YPD culture medium in which some glucose is substituted with ribose, and more preferably the YPD culture medium in which some glucose is substituted with ribose.

In a case where the microorganisms are Lactobacillus and Aspergillus, examples of the culture medium include No. 804 culture medium and the like, and are not limited thereto. The "No. 804 culture medium" herein is a culture medium obtained by dissolving 5 g of high polypeptone, 5 g of yeast extract, 5 g of glucose, and 1 g of MgSO₄·7H₂O in 1 L of water and sterilizing thereof.

A cultured and grown culture product of microorganisms may be used as it is.

Alternatively, the extract from the culture product of microorganisms may be prepared. Examples of the method of preparing an extract include an autolysis method of solubilizing a bacterial cell by using a proteolytic enzyme inherent in the bacterial cell, an enzymolysis method of solubilizing a bacterial cell by adding an enzyme preparation derived from microorganisms or plants, a hot water sampling method of solubilizing a bacterial cell by performing immersion for a certain time in hot water, an acid or alkali decomposition method of solubilizing a bacterial cell by adding various acids or alkali, a freezing and thawing method of crushing a bacterial cell by performing freezing and thawing one or more times, a physical crushing method of crushing a bacterial cell by physical stimulation, and the like. Examples of the physical stimulation used in the physical crushing method include ultrasonication, homogenization under a high pressure, grinding by mixing with a solid product such as glass beads, and the like.

Alternatively, the culture product of microorganisms may be subjected to dry processing to prepare a dry bacterial cell. Examples of the dry processing method include a freeze-drying method, a spray dry method, a drum dry method, and the like. In addition, by processing the obtained dry bacterial cell in a powder shape, it is possible to obtain dry bacterial powders excellent in handleability.

Alternatively, a fraction containing the polysaccharide may be prepared from the culture product of microorganisms. As the method of fractionating the fraction containing the polysaccharide from the culture product, it is possible to perform concentration and purification on an extract product into a fraction including a polysaccharide at a high concentration by fractionating the extract product obtained by extraction using hot water sampling and bacterial cell crushing (for example, the same method as the above-described extract preparation method and the like) using centrifugation, dialysis, an affinity column carrying a substance having high affinity with the polysaccharide and the like.

### «Dedifferentiation Inducer of Monocyte»

In one embodiment, the present invention provides a dedifferentiation inducer of monocytes including a polysaccharide containing a macrophage colony-stimulating factor and one or more selected from the group consisting of ribose, xylose, and derivatives thereof as an active ingredient.

According to the dedifferentiation inducer of the present embodiment, it is possible to dedifferentiate a monocyte and obtain a large amount of stem cells from a small amount of monocytes since the growth rate is high.

### <M-CSF>

In general, in the macrophage colony-stimulating factor (M-CSF), there are a membrane-binding type having a molecular weight of approximately 22,000 and a secretion type having a molecular weight of approximately 42,000. By disulfide bond, there are a membrane-binding type having a molecular weight of approximately 45,000 (dimer of 22,000), a secretion type having a molecular weight of approximately 85,000 (dimer of 42,000), and a high molecular weight type in which proteoglycan is further bound to the secretion type having a molecular weight of approximately 85,000 (dimer of 42,000). In the dedifferentiation inducer of the present embodiment, any of these may be used. Among these, the M-CSF used in the dedifferentiation inducer of the present embodiment is preferably the secretion type having a molecular weight of approximately 42,000, the secretion type having a molecular weight of approximately 85,000 (dimer of 42,000), or the high molecular weight type in which proteoglycan is further bound to the secretion type having a molecular weight of approximately 85,000 (dimer of 42,000).

In addition, the M-CSF may be any M-CSF as long as the M-CSF is derived from the same animal as that of the used monocyte, and is preferably a mammal. Examples of the mammal include human, horse, cow, monkey, chimpanzee, pig, sheep, rabbit, mouse, rat, dog, cat, and the like, and are not limited thereto. Among these, the mammal is preferably primate such as human, monkey, and chimpanzee, and is particularly preferably human.

As the M-CSF, a natural product may be purified and used, and those prepared by gene recombination may be used. As those prepared by gene recombination, it is determined that as long as the M-CSF has amino acids from at least N terminal to area of the 153rd position, the M-CSF has the same degree of specific activity as that of a natural type, and examples thereof include a recombinant of E. coli expression not having such a sugar chain.

### <Polysaccharide>

Examples of the polysaccharide used in the dedifferentiation inducer of the present embodiment include the same as exemplified in the above-described "Lin28a activator".

### <Monocyte>

In the present embodiment, the "monocyte" means a monocytic cell (including monocyte, mononucleosis, and monoblast) having a M-CSF receptor (Colony-stimulating factor-1 receptor; c-fms). In a case of using the mononucleosis, since only the monocyte contained therein grows, the mononucleosis may be used as a subject cell of dedifferentiation.

The monocyte in the present embodiment is preferably derived from a mammal. Examples of the mammal include human, horse, cow, monkey, chimpanzee, pig, sheep, rabbit, mouse, rat, dog, cat, and the like, and are not limited thereto. Among these, the mammal is preferably primate such as human, monkey, and chimpanzee, and is particularly preferably human.

In addition, the monocyte can be obtained from bone marrow, blood, and the like, and can be obtained with low invasiveness. Therefore, the monocyte is preferably obtained from blood, and particularly preferably obtained from peripheral blood.

As the method of separating a monocyte from a biological sample such as blood and purifying thereof, a known method may be used, and examples thereof include a method of separating a mononucleosis using a hemocyte separation solution Lymphoprep (registered trademark) (manufactured by Cosmo Bio Corporation), a method of separating a monocyte from the separated mononucleosis using antibody magnetic beads (manufactured by Miltenyi Biotech Corporation) recognizing surface antigen of CD14, and the like.

In addition, in a case of a human monocyte, a commercially available product may be used, and examples of the commercially available product include PT038 (manufactured by LONZA Corporation).

### <Method of Dedifferentiating Monocyte>

It is possible to dedifferentiate a monocyte, and grow and obtain a stem cell at a high growth rate by culturing a monocyte using a culture medium including the dedifferentiation inducer of the present embodiment.

A concentration of M-CSF contained in the culture medium is preferably equal to or more than 5 ng/mL and equal to or less than 100 ng, and more preferably 25 ng/mL.

In addition, a concentration of the polysaccharide contained in the culture medium is preferably equal to or more than 1 µg/mL and equal to or less than 300 µg/mL.

The culture medium used in culturing the monocyte may be a base culture medium including ingredients (inorganic salt, carbohydrate, hormone, essential amino acid, non-essential amino acid, vitamin) required for survival and growth of the monocyte and the like, and can be appropriately selected from those derived from the monocyte. Examples of the culture medium include Dulbecco's Modified Eagle's Medium (DMEM), Minimum Essential Medium (MEM), RPMI-1640, Basal Medium Eagle (BME), Dulbecco's Modified Eagle's Medium: Nutrient Mixture F-12 (DMEM/F-12), Glasgow Minimum Essential Medium (Glasgow MEM), and the like. In addition, as a serum ingredient, for example, FBS/FCS (Fetal Bovine/Calf Serum), NCS (Newborn Calf serum), CS (Calf Serum), HS (Horse Serum), and the like may be added by about 1 to 20%.

As the culture condition of the monocyte, culturing may be performed in the presence of 5% of carbon dioxide at 37°C for 7 days to 14 days. Regarding whether obtained cells are dedifferentiated stem cells or not, an expression amount of an undifferentiated marker shown in the "stem cell" to be described later may be checked using RT-PCR and the like.

### <Stem Cell>

In addition, in the present specification, the "stem cell" is a cell that expresses an undifferentiated marker and has self-replication ability. The stem cell obtained using the dedifferentiation inducer of the present embodiment can be prepared in a large amount from a monocyte. In addition, the stem cell can induce differentiation and preferably has pluripotent differentiation ability. In the stem cell obtained by using the dedifferentiation inducer of the present embodiment, CD14 and CD45 are positive.

Examples of the features of the stem cell obtained by using the dedifferentiation inducer of the present embodiment include significantly strong expression of CXCR4 gene. In addition, it is exemplified that at least one kind of Nanog, Nestin, c-Kit, CD9, and Oct3/4, preferably at least two kinds thereof, more preferably at least three kinds thereof, further more preferably at least four kinds thereof, and particularly preferably all genes of Nanog, Nestin, c-Kit, CD9, and Oct3/4 are expressed.

Large difference between the stem cell obtained by using the dedifferentiation inducer of the present embodiment and other dedifferentiated stem cell is strong expression of CXCR4 gene relating to homing operation of cells. In the stem cell obtained by using the dedifferentiation inducer of the present embodiment, CXCR4 gene is significantly strongly expressed compared to the stem cell obtained by culturing a monocyte in the presence of M-CSF alone.

More specifically, in the stem cell obtained by using the dedifferentiation inducer of the present embodiment, an expression amount of the CXCR4 gene is three times or more, particularly four times or more with respect to an expression amount in the stem cell obtained by culturing a monocyte in the presence of M-CSF alone, M-CSF + IL - 3, M-CSFF + IL - 6 + LIF, and the like, by analyzing with RT-PCR.

In addition, in the stem cell obtained y using the dedifferentiation inducer of the present embodiment, CXCR4 gene is significantly strongly expressed even compared to mesenchymal stem cell derived from bone marrow. Specifically by analyzing with RT-PCR, the expression amount of CXCR4 gene is two times or more, and preferably three times or more with respect to the expression amount in the mesenchymal stem cell derived from bone marrow.

It is determined that ligand SDF1 to a CXCR4 receptor is expressed in a damaged tissue at the time of fracture, a circulatory system disease, or a damaged site of a nerve tissue. For this reason, stronger expression of SDF1 is effective for providing a stronger homing effect of the stem cell obtained by using the dedifferentiation inducer of the present embodiment to a damaged site, from a viewpoint of a cell medicine.

The stem cell obtained by using the dedifferentiation inducer of the present embodiment can be used in treatment of diseases by being directly administered or injected to a disease site. It is preferable to culture and grow the stem cell in an appropriate culture medium before directly administering or injecting the stem cell to a disease site and directly administer or inject the stem cell to the disease site. As the culture medium of the stem cell, a general culture medium for cell culturing may be used, or a dedifferentiation inducing culture medium may be used.

Examples of the culture medium include the same as exemplified in the above-described "method of dedifferentiating monocyte". In addition, the dedifferentiation inducing culture medium may be a culture medium not including an extracellular matrix, a growth factor, cytokine, and the like required for maintaining differentiation, that is, a serum-free culture medium not including serum containing an unknown ingredient is preferable. Examples of the serum-free culture medium include UltraCULTURE (registered trademark) serum-free culture medium (manufactured by LONZA Corporation) and the like, and are not limited thereto.

Examples of the diseases that can be treated using the stem cell obtained by the dedifferentiation inducer of the present embodiment include external injury, inflammatory disease, damage in bone or cartilage, circulatory disease, nerve disease, liver disease, kidney disease, diabetes, atopic dermatitis, graft versus host disease (GVHD), and the like.

Examples of the inflammatory disease include pancreatitis, radiation damage, dermatomyositis, polymyositis, necrotizing fasciitis, chronic bronchitis, and the like.

Examples of the damage in bone or cartilage include fracture, osteoporosis, osteochondral fracture, osteochondritis, and the like.

Examples of the circulatory disease include dilated cardiomyopathy, myocardial infarction, ischemic cardiomyopathy, heart failure, myocardial hypertrophy, congestive heart failure, restenosis, arrhythmia, atherosclerosis, vasculitis, and the like.

Examples of the nerve disease include peripheral neuropathy, neuropathic pain, stroke, encephalitis, meningitis, diabetic neuropathy, attention deficit disorder, autism, Alzheimer's disease, Parkinson's disease, Creutzfeldt-Jakob's disease, brain trauma, spinal cord trauma, cerebral ischemia, and the like.

Examples of the liver disease include liver cirrhosis, chronic hepatitis, and the like.

Examples of the kidney disease include chronic renal failure, glomerular nephritis, renal ischemia, and the like.

«Therapeutic Agent»

In one embodiment, there is provided a therapeutic agent for diseases relating to damage in cells, tissues, or organs containing a polysaccharide including one or more selected from the group consisting of ribose, xylose, and derivatives thereof, as an active ingredient.

According to the therapeutic agent of the present embodiment, it is possible to effectively treat diseases relating to damage in cells, tissues, or organs with low invasiveness compared to surgery including transplantation or cell medical care.

By administering the therapeutic agent of the present embodiment into a living body, polysaccharides contained in the therapeutic agent reach the disease site, induces expression of Lin28a in the disease site, and activates Lin28a. With this, it is possible to repair damage in cells, tissues, or organs. In addition, the polysaccharide dedifferentiates monocytes into stem cells in cooperation with M-CSF already existing in the living body, the stem cells reach the disease site, and thereby it is possible to treat various diseases.

In addition, by administering the therapeutic agent of the present embodiment to the damaged cells, tissues, or organs in the in-vitro system, the polysaccharides contained in the therapeutic agent induce expression of Lin28a in the damaged cells and activates Lin28a. With this, it is possible to repair damage in cells, tissues, or organs in the in-vitro system.

In the present specification, examples of the "cell" include reproductive cell (sperm, egg, and the like), somatic cell constituting a living body, stem cell, precursor cell, cell which is separated from a living body, acquires immortalization ability, is stabilized and maintained outside the living body (cell line), cell which is separated from a living body and artificially genetically modified, cell which is separated from a living body and in which nucleus is artificially exchanged, and the like, and are not limited thereto.

Examples of the somatic cell constituting a living body include cells collected from an optional tissue such as skin, kidney, spleen, adrenal gland, liver, lung, ovary, pancreas, uterus, stomach, colon, small intestine, large intestine, urinary bladder, prostate, testicle, thymic gland, muscle, connective tissue, bone, cartilage, vascular tissue, blood, heart, eye, brain, nerve tissue, and are not limited thereto. More specific examples of the somatic cells include fibroblast, bone marrow cells, immune cells, red blood cells, thrombocyte, bone cells, bone marrow cells, pericyte, dendritic cells, epidermal keratinocyte, adipocyte, mesenchyme cells, epithelial cells, epidermal cells, interendothelial cells, vascular endothelial cells, lymphatic endothelial cells, liver cell, islet cells, cartilage cells, cumulus cells, glia cells, nerve cells (neuron), oligodendrocyte, microglia, macroglia, myocardial cells, esophagus cells, muscle cells, melanocyte, mononuclear cells, and the like, and are not limited thereto.

Examples of the immune cell include B lymphocyte, T lymphocyte, neutrophils, macrophage, monocyte, and the like.

Examples of the islet cell include α cells, β cells, δ cells, ε cells, PP cells, and the like.

Examples of the muscle cell include smooth muscle cells, skeletal muscle cells, and the like.

Examples of the stem cell include embryonic stem cells (ES cell), embryonic tumor cells, embryonic reproductive stem cells, induced pluripotent stem cells (iPS cell), nerve stem cells, hematopoietic stem cells, mesenchymal stem cells, liver stem cells, pancreas stem cells, muscular stem cells, reproductive stem cells, intestinal stem cells, cancer stem cells, hair follicle stem cells, and the like, and are not limited thereto.

The precursor cell is a cell which is in a stage of being differentiated into a specific somatic cell or reproductive cell from the stem cell.

The cell line is a cell which acquires limitless growth ability by artificial operation outside a living body. Examples of the cell line include HCT116, Huh7, HEK293 (human embryonic kidney cell), HeLa (human uterine cervix cancer cell line), HepG2 (human liver cancer cell line), UT7/TPO (human leukemia cell line), CHO (Chinese hamster ovary cell line), MDCK, MDBK, BHK, C-33A, HT-29, AE-1, 3D9, Ns0/1, Jurkat, NIH3T3, PC12, S2, Sf9, Sf21, HighFive, Vero, and the like, and are not limited thereto.

In the present specification, the "tissue" indicates a unit of structure gathering in a pattern based on a lineage in which one kind of stem cell is being differentiated, and has one role as a whole. For example, the epidermal keratinocyte is differentiated into a cell in which a stem cell present on a bottom layer of epiderm, terminally differentiated, forms a stratum corneum, and thereby exhibits a barrier function as an epiderm. Therefore, by using the therapeutic agent of the present embodiment, it is possible to construct a multicellular structure including one kind of cell derived from one cell lineage and regenerate epithelial tissue, connective tissue, muscular tissue, nerve tissue, and the like, for example.

In the present specification, the "organ" is constituted of two or more kinds of tissues and carries one function as a whole. Therefore, by using the therapeutic agent of the present embodiment, it is possible to construct a multicellular structure including at least two kinds of cells having different cell lineages and regenerate stomach, intestine, liver, kidney, and the like, for example.

Examples of the diseases relating to damage in cells, tissues, and organs to which the therapeutic agent of the present embodiment is applicable include the same as those exemplified in the above-described "Dedifferentiation Inducer".

### <Dosage and Administration Route>

A dosage of the therapeutic agent of the present embodiment is appropriately adjusted considering the age, the gender, the weight, the symptom, the administration method, treatment time, and the like of a subject animal (preferably mammal, more preferably primates, and particularly preferably human).

It is considered that an effective amount of the polysaccharide contained in the therapeutic agent of the present embodiment is equal to or more than 100 ng and equal to or less than 100 mg, preferably equal to or more than 1 µg and equal to or less than 10 mg, and more preferably equal to or more than 10 µg and equal to or less than 5 mg, per 1 kg of a weight of a human adult.

The therapeutic agent of the present embodiment can be administered 1 to 4 times a day.

Examples of the administration form include intraarterial injection, intravenous injection, subcutaneous injection, an intranasally, transbronchially, intramuscularly, percutaneously or orally known method to those skilled in the art, and the like.

### <Composition>

The therapeutic agent of the present embodiment may include a pharmaceutically acceptable carrier or diluent, in addition to the above-described therapeutically effective amount of polysaccharide. Examples of the pharmaceutically acceptable carrier or diluent include excipient, diluent, extender, disintegrator, stabilizer, preservative, buffer, emulsifier, perfuming agent, colorant, sweetener, viscous agent, flavoring agent, solubilizer, additive, and the like. By using one or more carriers, it is possible to prepare a therapeutic agent in the form of tablet, pill, powder, liquid, suspension, emulsion, granule, capsule, suppository, injection (liquid, suspension, and the like), ointment, and the like.

In addition, a colloidal dispersion type can be used as a carrier. The colloidal dispersion type is expected to have an effect of enhancing in-vivo stability of the above-described polysaccharide, or an effect of enhancing transferability of the above-described polysaccharide to a specific organ, tissue, or cell. Examples of the colloidal dispersion type include polyethylene glycol, polymer composite, polymer aggregate, nanocapsule, microsphere, beads, oil-in-water emulsifier, micelle, mixed micelle, lipid containing liposome, and the like, and is preferably liposome or artificial membrane vesicle having an effect of efficiently transferring the above-described polysaccharide to a specific organ, tissue, or cell.

Examples of the preparation of the therapeutic agent of the present embodiment include those orally used as tablet sugar-coated depending on the necessity, capsule, elixir, and microcapsule.

Alternatively, examples of the preparation of the therapeutic agent of the present embodiment include an aseptic solution of water or a pharmaceutically acceptable liquid other than water or those parenterally used in the form of injection of a suspension. In addition, the examples include those prepared by being appropriately combined with the pharmaceutically acceptable carrier or diluent, more specifically, sterilized water or physiological saline, plant oil, emulsifier, suspension, surfactant, stabilizer, perfuming agent, excipient, vehicle, antiseptic, binder, and the like, and admixed in a unit dosage form required for generally recognized pharmaceutical implementation.

In a case where the preparation unit form is tablet or capsule, examples of the additive that can be admixed include binder, excipient, extender, lubricant, sweetener, perfuming agent, and the like.

Examples of the binder include gelatin, corn starch, gum tragacanth, gum Arabic, and the like.

Examples of the excipient include crystalline cellulose and the like.

Examples of the extender include corn starch, gelatin, alginic acid, and the like.

Examples of moistening agent include magnesium stearate and the like.

Examples of the sweetener include sucrose, lactose, saccharine, and the like.

Examples of the perfuming agent include peppermint oil, Gaultheria adenothrix, and the like.

In addition, in a case where the preparation unit form is capsule, it is possible to contain a liquid carrier such as fat and oil in addition to the above materials.

In a case where the preparation unit form is injection, it is possible to perform prescription according to common pharmaceutical implementation using a vehicle such as distilled water for injection. In addition, as an aqueous solution for injection, an isotonic solution and an appropriate solubilizer or nonionic surfactant may be used in combination.

The isotonic solution may include physiological saline, dextrose, or other adjuvants. Examples of the adjuvants include D-sorbitol, D-mannose, D-mannitol, sodium chloride, and the like.

Examples of the solubilizer include alcohol, polyalcohol, and the like. Specific examples of the alcohol include ethanol and the like. Specific examples of the polyalcohol include propylene glycol, polyethylene glycol, and the like.

Examples of the nonionic surfactant include polysorbate 80 (TM), HCO-50, and the like.

In addition, in a case where the preparation unit form is injection, it is possible to contain an oily liquid in addition to the above materials.

Examples of the oily liquid include vegetable oil such as sesame oil, soybean oil, and olive oil.

In addition, a solubilizer may be further used in combination. Examples of the solubilizer include benzyl benzoate, benzyl alcohol, and the like.

In addition, a buffer, an analgesic agent, a stabilizer, or an anti-oxidant may be further mixed.

Examples of the buffer include a phosphate buffer solution, a sodium acetate buffer solution, and the like.

Examples of the analgesic agent include procaine hydrochloride and the like.

Examples of the stabilizer include benzyl alcohol, phenol, and the like.

In addition, in a case where the preparation unit form is injection, the injection can be prepared as a suspension or opacifier.

The injection prepared in such a manner is generally filled in an appropriate ample.

In addition, sterilization of such injection can be performed by filtration sterilization using a filter, mixing of a sterilizer, and the like. Alternatively, the injection can be prepared as a form of extemporaneously preparation. That is, it is possible to use a polysaccharide by making the polysaccharide as a sterile solid composition by the freeze-drying method and the like and dissolving thereof in distilled water for injection or another solvent before use.

### <Treatment Method>

An aspect of the present invention is to provide a therapeutic agent including the above-described polysaccharide for treating diseases relating to damage in cells, tissues, and organs.

In addition, an aspect of the present invention is to provide a therapeutic agent including a therapeutically effective amount of the above-described polysaccharide and a pharmaceutically acceptable carrier or diluent.

In addition, an aspect of the present invention is to provide use of the above-described polysaccharide for preparing a therapeutic agent for diseases relating to damage in cells, tissues, or organs.

In addition, an aspect of the present invention is to provide a treatment method for diseases relating to damage in cells, tissues, or organs, including administering an effective amount of the above-described polysaccharide to a patient who needs treatment.

### <<Food or Beverage Product>>

In one embodiment, the present invention provides a food or beverage product for preventing or alleviating diseases relating to damage in cells, tissues, or organs including a polysaccharide containing one or more selected from the group consisting of ribose, xylose, and derivatives thereof.

According to the food or beverage product of the present embodiment, it is possible to effectively prevent or alleviate diseases relating to damage in cells, tissues, or organs. In addition, from a viewpoint of safety, consuming the polysaccharide has been experienced, and it is considered that it is not problematic to consume the polysaccharide on a daily basis. Therefore, it is possible to provide a safe and effective food or beverage product.

In the present specification, the "food or beverage product" is a combination of food and beverage and mainly means a processed food. In addition, the food or beverage product of the present embodiment include health food (including specific health food), functional food, health beverage, and functional beverage.

Examples of the diseases relating to damage in cells, tissues, or organs that can be prevented or alleviated by the food or beverage product of the present embodiment include the same as exemplified in the above-described "dedifferentiation inducer".

The form of the food or beverage product of the present embodiment may be solid or may be liquid, and the above-described polysaccharide can be widely used by being added to general foods as an additive. Specific examples of the kind of the food or beverage product include beverage (including concentrated crude liquid and powder for preparation of beverage), noodles, confectioneries, fishery or livestock processed food, dairy products, oils and fats and oil processed foods, condiments, soups, stew, curry, bread, jam, salad, daily dishes, pickles, and the like, and are not limited thereto.

More specific examples of the beverage include cool beverages, alcoholic beverages, and the like.

Examples of the cooling beverage include mineral water, carbonated beverages, nutrient beverages, sports beverages, cocoa beverages, fruit beverages, milk, coffee, tea, soybean milk, vegetable beverages, alcoholic taste beverages, and the like.

Examples of the alcoholic taste beverage include non-alcoholic beer, non-alcoholic wine, and the like.

Examples of the alcohol include beer, sparkling wine, cocktail, chuhai, shochu, sake, whiskey, brandy, wine, and the like.

Examples of the noodles include buckwheat noodles, udon noodles, spaghetti, gelatin noodles, coating doughs for jiaozi, coating doughs for siomai, Chinese noodles, instant noodles, and the like.

Examples of the confectionery include sweets, chewing gum, candy, gummy, gum, caramel, chocolate, tablet confectioneries, snack confectioneries, baked confectioneries, jelly, pudding, cold confectioneries, and the like.

Examples of the baked confectioneries include biscuit, cake, madeleine, and the like.

Examples of the cold confectionery include ice cream, ice sherbet, shaved ice, and the like.

Examples of the fishery or livestock processed food include kamaboko, hamburger, ham, sausage, and the like.

Examples of the dairy product include processed milk, fermented milk, yogurt, butter, cheese, fresh cream, and the like.

Examples of the oil and fat and oil processed food include salad oil, tempura oil, margarine, mayonnaise, shortening, whipped cream, dressing, and the like.

Examples of the condiment include sauce, tare, and the like.

In addition, in the food or beverage product of the present embodiment, by processing the above-described polysaccharide into soft capsule, hard capsule, tablet, powder, and the like, it is possible to consume the polysaccharide as a supplement and the like.

The food or beverage product of the present embodiment may be appropriately mixed with a commonly used additive depending on the type. Examples of the additive include sweeteners, acidulant, excipient, binders, diluent, perfume, buffers, thickeners, gelling agents, colorants, stabilizers, emulsifiers, dispersants, suspending agents, antiseptics, and the like.

Examples of the sweetener include sugar, fructose, isomerized liquid sugar, glucose, aspartame, stevia, and the like.

Examples of the acidulant include citric acid, malic acid, tartaric acid, and the like.

Examples of the excipient include dextrin, starch, and the like.

A mixture amount of the polysaccharide in the food or beverage product of the present embodiment may be an amount in which the physiological effect or pharmacological effect can be exhibited, and considering the dosage in the above-described "therapeutic agent" and the general consumption amount of the subject food or beverage product, the amount may be generally equal to or more than 100 ng and equal to or less than 100 mg, preferably equal to or more than 1 µg and equal to or less than 10 mg, and more preferably equal to or more than 10 µg and equal to or less than 5 mg, per 1 kg of a weight of a human adult. For example, in a case of the solid food, the amount may be equal to or more than 10 to equal to or less than 50 weight%, and in a case of a liquid food such as a beverage, the amount may be equal to or more than 0.1 and equal to or less than 10 weight%.

### «Cosmetic Product»

In one embodiment, the present invention provides a cosmetic product including a polysaccharide containing one or more selected from the group consisting of ribose, xylose, and derivatives thereof.

The cosmetic product of the present embodiment has excellent anti-inflammatory effect, whitening effect, and anti-aging effect. In addition, since the above-described polysaccharide is harmless, it is not problematic to percutaneously consume the product on a daily basis. Therefore, it is possible to provide a safe and effective cosmetic product.

In the present specification, the "inflammation" means local defense reaction caused by the immune system triggered by invasion of foreign matter into a living body, tissue disorder, or the like. In the inflammatory reaction, there is a case where the living body helps exclusion of the non-self, whereas certain damage is caused even in the living body which is the self. By applying the cosmetic product of the present embodiment, the polysaccharide contained in the cosmetic product activates Lin28a, damage in cells and the like caused by the inflammation can be repaired, and the inflammation can be relieved.

In the present specification, the "whitening" means prevention or improvement of pigmentation, blotches, freckles, chloasma, and the like of skin. By applying the cosmetic product of the present embodiment, the polysaccharide contained in the cosmetic product activates Lin28a, accelerates metabolism of cells in the skin, and thereby it is possible to prevent or improve pigmentation, blotches, freckles, chloasma, and the like of the skin.

In the present specification, the "aging" means that there are age-related skin changes, specifically, various unpreferable changes in terms of beauty that "wrinkles in the skin increase", "the skin becomes flabby", and "resilience of the skin is lowered".

In addition, the "wrinkle" is a change in the shape of the skin that is expressed as clear lines compared to the texture of the skin constituted of sulci cutis and cristae cutis, and means one of the most prominent changes in which skin aging is expressed in the appearance. The wrinkles appear in the face, the neck, and other body parts, and as the wrinkles are increased and deepen, this makes the person aged. This is essentially distinguished from rough skin accompanying partial peeling on the stratum corneum or partial disappearance of cristae cutis due to drying and the like or pimpled face in terms of morphology.

By applying the cosmetic product of the present embodiment, it is possible to "prevent wrinkles", that is, to suppress formation of wrinkles in the skin that is liable to be exposed to an environment in which wrinkles are easily formed, that is, a dry environment and the like, in advance. In addition, by applying the cosmetic product of the present embodiment, it is possible to "improve formed wrinkles", that is, to significantly reduce the length, the depth, the number, and the like of wrinkles formed by being exposed to an environment in which wrinkles are easily formed, that is, a dry environment and the like.

The presence or absence of such "prevent wrinkles" and "improve formed wrinkles" can be determined by quantitative or qualitative means, and can be determined by a double shielded pair comparison method (ICH Guideline E9) in order to enhance the accuracy.

The mixture amount of the polysaccharide contained in the cosmetic product of the present embodiment may be an amount in which the physiological effect or pharmacological effect can be exhibited, and considering the dosage in the above-described "therapeutic agent", the amount may be generally equal to or more than 100 ng and equal to or less than 100 mg, preferably equal to or more than 1 µg and equal to or less than 10 mg, and more preferably equal to or more than 10 µg and equal to or less than 5 mg, per 1 kg of a weight of a human adult.

The cosmetic product of the present embodiment is preferably used by being applied in the exoderm. The application method is not particularly limited. For example, the cosmetic product may be applied in an appropriate amount depending on the skin area to be applied, one time or more a day, for example, one time or more and three times or less a day, in an amount of equal to or more than 1 mL and equal to or less than 5 mL per time.

The form of the cosmetic product of the present embodiment is not particularly limited, and may be an optional form such as solution, paste, gel, solid, and powder. Specific examples of the cosmetic product include skin care cosmetic product, makeup cosmetic product, body cosmetic product, perfume cosmetic product, dentifrice, soap, aerosol, bathing agent, hair tonic, sun-screening agent, and the like.

Examples of the skin care cosmetic product include cleansing foam, cleansing oil, cleansing gel, skin lotion, lotion, cream, emulsion, essence, gel, pack, and the like.

Examples of the makeup cosmetic product include foundation, eyeliner, eyebrow pencil, mascara, lipstick, face powder, powder, and the like.

Examples of the body cosmetic product include shampoo, hair rinse, hair conditioner, enamel, and the like.

Examples of the perfume cosmetic product include perfume, eau de cologne, and the like.

In the cosmetic product of the present embodiment, various ingredients generally used in cosmetic products may be mixed, in addition to the polysaccharide, within a range not inhibiting the effect of the present embodiment.

Examples of the various ingredients generally used in cosmetic products include oily ingredients, surfactants, amino acids, amino acid derivatives, lower alcohols, polyhydric alcohols, sugar alcohols and alkylene oxide adducts thereof, water-soluble polymers, sterilizers, anti-fungal agents, anti-inflammatory agents, analgesic agents, anti-mycotic agents, keratin softening and releasing agents, skin colorants, hormonal agents, ultraviolet absorbing agents, hair tonics, whitening agents, anti-perspirants, astringent active ingredients, sweat deodorants, vitamin preparations, vasodilators, crude drugs, pH regulators, viscosity modifiers, pearling agents, natural perfumes, synthetic perfumes, pigments, anti-oxidants, antiseptic agents, emulsifiers, fats, wax, silicone compounds, perfumed oil, and the like.

Examples of the oily ingredient include saturated or unsaturated fatty acids, higher alcohols obtained therefrom, and the like. Specific examples of the oily ingredients include esters of straight or branched fatty alcohols, squalane, squalene, castor oil, hydrogenated castor oil and derivatives thereof, glycerides, beeswax, lanolins (including liquid lanolin and purified lanolin) and derivatives thereof, animal and plant-derived oily raw materials, petroleum and mineral-derived oily raw materials, silicones, resin acids, fatty acid esters, ketones, and the like.

Examples of the esters of straight or branched fatty alcohol include myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl erucate, behenyl myristate, behenyl palmitate, behenyl stearate, behenyl isostearate, behenyl oleate, behenyl behenate, behenyl erucate, ersil myristate, ersil palmitate, ersil stearate, elcyl isostearate, ersil oleate, ersil behenate, ercyl erucate, and the like.

Examples of the glycerides include hydroxystearic acid monoglyceride, hydroxystearic acid diglyceride, isostearic acid monoglyceride, isostearic acid diglyceride, oleate monoglyceride, oleate diglyceride, ricinoleic acid monoglyceride, ricinoleic acid diglyceride, linoleic acid monoglyceride, linoleic acid diglyceride, linolenic acid monoglyceride, linolenic acid diglyceride, tartaric acid monoglyceride, diglyceride tartrate, monoglyceride citrate, diglyceride citrate, malic acid monoglyceride, diglyceride malate, 1 to 30 mol ethylene oxide adduct of the glycerides, and the like.

Examples of the animal and plant-derived oily raw material include almond oil, avocado oil, olive oil, rapeseed oil, coconut oil, macadamia nuts oil, jojoba oil, carnauba wax, sesame oil, cacao oil, palm oil, mink oil, Japanese wax, candelilla wax, spermaceti wax, and the like.

Examples of the petroleum and mineral-derived oily raw materials include paraffin, microcrystalline wax, liquid paraffin, petrolatum, ceresin, and the like.

Examples of the silicons include methylpolysiloxane, polyoxyethylene-methyl polysiloxane, polyoxypropylene-methyl polyoxysiloxane, poly (oxyethylene, oxypropylene)-methyl polysiloxane, methylphenyl polysiloxane, fatty acid-modified polysiloxane, aliphatic alcohol-modified polysiloxane, amino acid-modified polysiloxane, and the like.

Examples of the surfactant include anionic surfactant, nonionic surfactant, cationic surfactant, amphoteric surfactant, and the like.

Examples of the anionic surfactant include N-long chain acylaminate, N-long chain fatty acid acyl-N-methyltaurine salt, alkyl sulfate and alkyl oxide adducts thereof, fatty acid amide ether sulfate, metal salts and weak base salts of fatty acid, sulfosuccinic acid-based surfactants, alkyl phosphate and alkylene oxide adduct thereof, alkyl ether carboxylic acids, and the like.

Examples of the N-long-chain acylaminate include N-long chain acyl acidic amino acid salt, N-long chain acyl neutral amino acid salt, and the like.

Examples of the N-long chain acyl acidic amino acid salt include N-long chain acylglutamate, N-long chain acylaspartate, and the like.

Examples of the N-long chain acyl neutral amino acid salt include N-long chain acylglycine salts, N-long chain acylalanine salts, N-long chain acylthreonine salts, and the like.

Examples of the nonionic surfactant include ether type surfactants, ester type surfactants, ether ester type surfactants, alkyl glucosides, cured castor oil pyroglutamic acid diester and ethylene oxide adducts thereof, nitrogen-containing nonionic surfactants, and the like.

Examples of the ether type surfactant include glycerol ethers and alkylene oxide adducts thereof, and the like.

Examples of the ester type surfactant include glycerol esters and alkylene oxide adducts thereof, polyoxyalkylene fatty acid esters, glycerol esters, fatty acid polyglycerol esters, acylamino acid polyglycerol esters, sorbitane esters, sucrose fatty acid esters, and the like.

Examples of the ether ester type surfactant include sorbitan ester and alkylene oxide adduct thereof, and the like.

Examples of the nitrogen-containing nonionic surfactant include fatty acid alkanolamide and the like.

Examples of the cationic surfactant include aliphatic amine salts and quaternary ammonium salts thereof, aromatic quaternary ammonium salts, fatty acid acylarginine esters, alkyloxy hydroxy propylarginine salts, and the like.

Examples of the aliphatic amine salt include alkylammonium chloride, dialkylammonium chloride, and the like.

Examples of the aromatic quaternary ammonium salt include benzalkonium salt and the like.

Examples of the amphoteric surfactant include betaine type surfactant, aminocarboxylic acid type surfactant, imidazoline type surfactant, and the like.

Examples of the betaine type surfactant include alkylbetaine, alkylamide betaine, aminopropionate, carboxybetaine, and the like.

Examples of the amino acids include glycine, alanine, serine, threonine, arginine, glutamate, aspartate, leucine, valine, and the like.

Examples of the amino acid derivatives include pyrrolidonecarboxylic acid and salts thereof, trimethylglycine, lauroylidine, and the like.

Examples of lower alcohol include ethanol, propanol, isopropanol, butanol, and the like.

Examples of the polyalcohol include glycerol, diglycerol, ethylene glycol, 1,3-butylene glycol, propylene glycol, isoprene glycol, and the like.

Sugar alcohol and alkylene oxide adduct thereof include mannitol, erythritol, and the like.

Examples of the water-soluble polymer include polyaminic acid and salts thereof, polyethylene glycol, Arabic acid, alginic acid, xanthanum, hyaluronic acid, hyaluronic acid salt, chitin, chitosan, water-soluble chitin, carboxyvinyl polymers, carboxymethyl cellulose, hydroxyethyl cellulose, polyacrylamide, polyvinyl alcohol, polyvinylpyrrolidone, hydroxypropyltrimethylammonium chloride, polydimethylmethylenepiperidium chloride, polyvinylpyrrolidone derivative quaternary ammonium, cationized proteins, collagen decomposition products and derivative thereof, acylated proteins, polyglycerol, and the like.

Examples of the polyamino acid include polyglutamic acid, polyaspartic acid, and the like.

Examples of the sterilizer and anti-fungal agent include 4-hydroxybenzoic acid, salts and esters thereof, trichlorosan, chlorhexidine, phenoxyethanol, mentol, mint oil, glyceryl caprinate, glyceryl caprylate, salicylic acid-N-alkylamide, and the like.

Examples of the anti-inflammatory agent, analgesic agent, anti-mycotic agent, keratin softening and releasing agent, skin colorant, and hormonal agent include hinokitiol, hydrocortisone (V), ε-aminocarboxylic acid, azulene, allantoin, glycyrrhizic acid derivative, β-glycyrrhetinic acid, and the like.

The ultraviolet absorbing agent is an organic substance (light protection filter) that is liquid or crystal at room temperature, absorbs ultraviolet rays, and can discharge the absorbed energy as radiation of a longer wavelength (for example, heat), and examples of the ultraviolet absorbing agent include UV-B filter, UV-A filter, and the like.

The UV-B filter may be any of oil-soluble filter and water-soluble filter.

Examples of the oil-soluble substance of the UV-B filter include those shown in the following 1) to 9).
1) 3-benzylidene camphor or 3-benzylidene norcamphor and derivatives thereof (for example, 3-(4-methylbenzylidene)-camphor)
2) 4-aminobenzoic acid derivative (preferably, 4-(dimethylamino)-benzoic acid-2-ethylhexylester, 4-(dimethylamino)-benzoic acid-2-octyl ester, or 4-(dimethylamino)-benzoic acid pentyl ester)
3) Cinnamic acid ester (preferably, 4-methoxycinnamic acid 2-ethylhexyl ester, 4-methoxycinnamic acid propylester, 4-methoxycinnamic acid isopentyl ester, or 2-cyano-3,3-phenylcinnamic acid-2-ethylhexyl ester [octocrylene])
4) Salicylic acid ester (preferably, salicylic acid-2-ethylhexyl ester, salicylic acid-4-isopropylbenzyl ester, or salicylic acid homomenthyl ester)
5) Benzophenone derivative (preferably, 2-hydroxy-4-methoxybenzophenone, 2 -hydro xy-4-methoxy-4' -methylbenzophenone, or 2,2' -dihydroxy-4-methoxybenzophenone)
6) Benzalmalonate ester (preferably, 4-methoxybenzalmalonate di-2-ethylhexyl ester)
7) Triazine derivative (for example, 2,4,6-trianilino-(p-carbo-2'-ethyl-1'-hexyloxy)-1,3,5-triazine, octyl-triazone, or dioctyl-butamide-triazone [Uvasorb (registered trademark) HEB])
8) Propane-1,3-dione (for example, 1-(4-t-butylphenyl)-3-(4'-methoxyphenyl)-propane-1,3-dione)
9) Ketotricyclo (5.2.1.0) decane derivative
   Examples of the water-soluble substance of the UV-B filter include those shown in the following 10) to 12).
10) 2-phenylbenzimidazol-5-sulfonic acid and alkali metal salt thereof, alkaline earth metal salt, ammonium salt, alkylammonium salt, alkanolammonium salt, and glucanmonium salt
11) Sulfonic acid derivative of benzophenone and salt thereof (preferably, 2-hydroxy-4-methoxybenzophenone-5-sulfonic acid and salt thereof)
12) Sulfonic acid derivative of 3-benzylidene camphor and salt thereof (for example, 4-(2-oxy-3-bornylidene methyl)-benzenesulfonic acid and 2-methyl-5-(2-oxy-3-bornylidene)-sulfonic acid)

As the UV-A filter, in particular, a benzoylmethane derivative is used. Examples of the benzoylmethane derivative include 1-(4' -t-butylphenyl)-3 -(4' -methoxyphenyl)-propane-1,3 -dione, 4-t-butyl-4'-methoxydibenzoylmethane (Parsol (Registered trademark) 1789), 1-phenyl-3-(4'-isopropylphenyl)-propane-1,3-dione, enamine compounds, and the like.

Examples of the hair tonic include pantothenic acid and derivatives thereof, placenta extract, allantoin, and the like.

Examples of the whitening agent include anti-oxidant, tyrosinase activity inhibitor, α-MSH (a-melanocyte-stimulating hormone) antagonist, and the like.

Examples of the anti-oxidant include ascorbic acid and the like.

Examples of the tyrosinase activity inhibitor include kojic acid, arbutin, ellagic acid, rucinol, and the like.

Examples of the α-MSH antagonist include d-2-Nal-Arg-Leu-NH2 and the like.

Examples of the anti-perspirant, astringent active ingredient, sweat deodorant include salt of aluminum, zirconium, or zinc. Examples of the salt of aluminum, zirconium, or zinc include aluminum chloride, aluminum chlorohydrate, aluminum zirconium trichlorohydrate, aluminum zirconium tetrachlorohydrate, zinc pyrrolidone carboxylate, and the like.

Examples of the vitamin preparation include vitamin A, B₁, B₂, B₆, E, derivatives thereof, and the like.

Examples of the vasodilator include swertia herb extract, cephalantin, and the like.

Examples of the crude drug include apricot extract, avocado extract, aloe extract, turmeric extract, orange extract, chamomilla extract, kiwi extract, gingko extract, black tea extract, sage extract, swertia herb extract, tonin extract, rose extract, sunflower extract, grape extract, sponge gourd extract, peach leaf extract, eucalyptus extract, lavender extract, green tea extract, apple extract, lemon extract, rosemary extract, and the like.

Examples of the pH regulator include citric acid, adipic acid, ascorbic acid, phosphoric acid, glutamic acid, lactic acid, sulfuric acid, hydrochloric acid, ammonium, sodium hydroxide, potassium hydroxide, arginine, hydroquinone and derivatives thereof, γ-oryzanol, and the like.

Examples of the viscosity modifier include agar, organically modified clay minerals, and the like.

Examples of the pearling agent include alkylene glycol ester, fatty acid alkanolamide, fatty acid monoglyceride, fatty ether, and the like.

Examples of the natural perfume include plant raw material such as flower, stem and leaf, fruit, pericarp, root, tree, needleleaf and branch, resin, balsam, and animal raw material such as civet and beaver, and the like.

Examples of the flower include lily, lavender, rose, jasmin, neroli, ylang-ylang, and the like.

Examples of the stem and leaf include geranium, patchouli, petitgrain, tarragon, lemongrass, sage, thyme, and the like.

Examples of the fruit include anise, coriander, caraway, juniper, and the like.

Examples of the pericarp include bergamot, lemon, orange, and the like.

Examples of the root include nutmeg, angelica, celery, cardamon, costus, iris, sweet flag, and the like.

Examples of the tree include pine, sandalwood, lignum vitae, cedar, rose wood, and the like.

Examples of the needleleaf and branch include spruce, fir tree, pine, shrub pine, and the like.

Examples of the resin and balsam include galbanum, elemi, benzoin, myrrh, mastic, opopanax, and the like.

In addition, examples of the essential oil which is frequently used as an aromatic ingredient and has comparatively low volatility include sage oil, chamomile oil, clove oil, Melissa oil, mint oil, cinnamon leaf oil, lime oil, juniper berry oil, vetiver oil, mastic oil, galbanum oil, labdanum oil, lavandin oil, bergamot oil, dihydromyrcenol, lilial, lyral, citronellol, phenylethyl alcohol, α-hexylcinnamaldehyde, geraniol, benzylacetone, cyclamenaldehyde, linalool, Boisambrene Forte, Ambroxan, indole, Hedione, Sandelice, citrus oil, mandarin oil, orange oil, allyl pentyl glycolate, cyclovertal, labandin oil, clary oil, β-damascone, geranium oil bourbon, cyclohexyl salicylate, Vertofix Coeur, Iso-E-Super, Fixolide NP, Evanil, Iraldein gamma, phenylacetic acid, geranyl acetate, benzyl acetate, rose oxide, Romilat, Irotyl and Floramat, peppermint oil, spearmint oil, anise oil, star anise oil, caraway oil, eucalyptus oil, fennel oil, citrus oil, wintergreen oil, clove oil, menthol oil, and the like.

Examples of the synthetic perfume include ester, ether, aldehyde, ketone, alcohol, hydrocarbon-type perfume, and the like.

Examples of the pigment include Cochineal red A (C. I. 16255), patent blue (C. I. 42051), chlorophyllin (C. I. 75810), and the like.

Examples of the anti-oxidant include tocopherol, sodium sulfite, and the like.

Examples of the antiseptic agent include phenoxyethanol, paraben, pentanediol, and the like.

Examples of the emulsifier include nonionic surfactant and the like.

Examples of the fat and wax include 12-hydroxy stearic acid, lanolin, beeswax, candelilla wax, carnauba wax, and the like.

Examples of the silicone compound include dimethyl polysiloxane, methylphenyl polysiloxane, cyclic silicone, modified silicone compound (in liquid or resin form at room temperature), simethicone which is a mixture of dimethicone and hydrogenated silicate having an average chain length of 200 to 300 dimethyl siloxane units, and the like. The kinds of modification in the modified silicone compound include one or more selected from the group consisting of amino, fatty acid, alcohol, polyether, epoxy, fluorine, glycoside, and alkyl.

Examples of the perfumed oil include a mixture of natural perfume and synthetic perfume and the like. Examples of the natural and synthetic perfume are the same as those described above.

Each of the above-described various ingredients may be mixed alone, or may be mixed in combination of two or more ingredients.

### Examples

Hereinafter, the present invention will be described using examples, but the present invention is not limited to the following examples.

### [Example 1]

### (1) Preparation of Culture Product (Exocrine Secretion) of Saccharomyces boulardii

First, Saccharomyces boulardii was cultured with shake overnight at room temperature by using a 100 mL of YPD culture medium (containing 2% peptone, 1% yeast extract, 2% glucose). Subsequently, in the logarithmic growth phase, centrifugation (1,000 g, 15 minutes at room temperature) was performed, and a culture supernatant was recovered. Subsequently, powdered ammonium sulfate was added to the culture supernatant so as to be 75% saturated ammonium sulfate (516 g with respect to 1 L of sample), the resultant product was cooled in ice water for 1 or more hours to recover a precipitate by centrifugation (1,000 g, 15 minutes at 4°C). Subsequently, the recovered precipitate was dissolved in a small amount of distilled water, dialysis was performed against enough water using a dialysis membrane having a limit molecular weight of 3,500, and remaining ammonium sulfate was removed. After the dialysis, exocrine secretion of Saccharomyces boulardii was recovered by freeze-drying, and weight measurement was performed. In this manner, approximately 20 mg of exocrine secretion was obtained.

### (2) Preparation of Mouse Vascular Endothelial Cell Line TKD2 Cell

Mouse vascular endothelial cell line TKD2 cell (National Institute of Biomedical Innovation, Health, and Nutrition, distributed from JCRB Cell Bank, Cell number: IFO50374) was sown in advance in a 6-well plate to be 4 × 10⁴ cells/well, and cultured overnight at 33°C in 5% CO₂ environment.

### (3) Lin28a Expression Induction

Subsequently, on the day after the date when the mouse vascular endothelial cell line TKD2 cell was sown, the exocrine secretion of Saccharomyces boulardii prepared in (1) was added to the culture medium to reach a final concentration of 100 µg/mL, and cultured for 5 days while performing addition. After the addition, the culture medium was not exchanged at all. In addition, as a control, a cell not added with exocrine secretion was prepared, and as a positive control, a cell added with 6CiPSs (Reference: Hou P., et al, "Pluripotent stem cells induced from mouse somatic cells by small-molecule compounds", Science, vol. 341, Issue 6146, p651-654, 2013) which is a mixture of 6 small-molecule compounds capable of inducing iPS cell, instead of the exocrine secretion, was prepared. In addition, as a control group, a group obtained by adding a ganglioside (manufactured by Techno Chemical Corporation, 1060) or sweet potato-derived polysaccharide, instead of the exocrine secretion, to the culture medium such that a final concentration was 100 µg/mL was also prepared.

After culturing, the cell was isolated and recovered by using 0.25% of Trypsin-EDTA solution (manufactured by ThermoFisher Corporation, 25200056). Subsequently, total RNA was recovered from the recovered cell by using RNeasy Micro Kit (QIAGEN 74004). From the total RNA, cDNA was produced by using ImProm-II (registered trademark) reverse Transcription System (Promega A3801), and expression of Lin28a was monitored by PCR using primers shown in the following Table 1.

**[Table 1]**

| | Base Sequence (5' → 3') | SEQ No. |
|---|---|---|
| Forward primer | 5'-cagaagcgaagatccaaagg-3' | 1 |
| Reverse primer | 5'-caggctttccctgagaactg-3' | 2 |

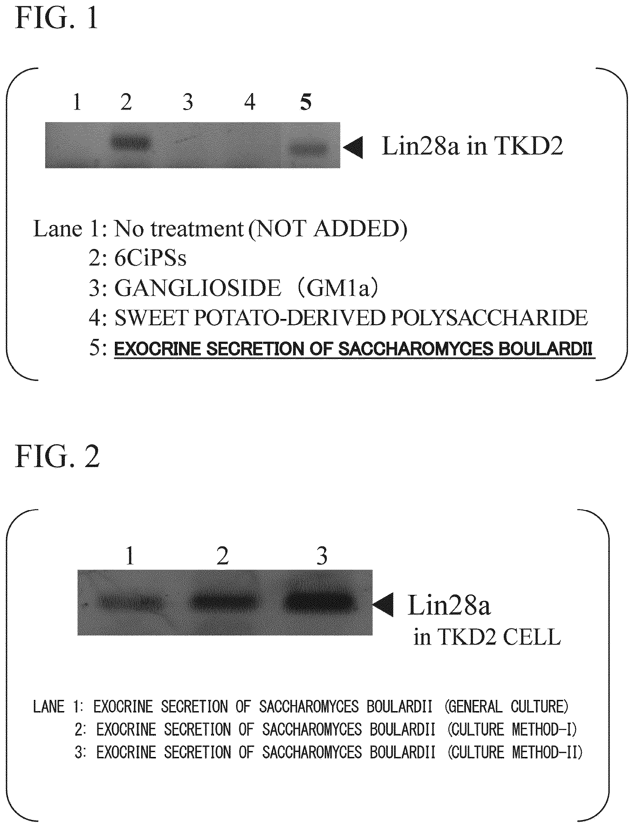

In addition, the reaction liquid after PCR was subjected to agarose gel electrophoresis, and the result was shown in FIG. 1.

From FIG. 1, in the TKD2 cell added with 6CiPSs of positive control, which is Lane 2, and the TKD2 cell added with exocrine secretion of Saccharomyces boulardii, which is Lane 5, it was checked that Lin28a was expressed.

On the other hand, in the TKD2 cell added with ganglioside and sweet potato-derived polysaccharide, expression of Lin28a was not induced.

### [Example 2]

### (1) Preparation of Culture Product (Exocrine Secretion) of Saccharomyces boulardii

First, Saccharomyces boulardii was cultured with shake overnight at room temperature by using a YPD culture medium (containing 2% peptone, 1% yeast extract, 2% glucose) (hereinafter, referred to as "General Culture" in some cases), a YPD culture medium (containing 2% peptone, 1% yeast extract, 0.2% glucose, 1.8% rhamnose) in which some glucose was substituted with rhamnose) (hereinafter, referred to as "Culture Method-I" in some cases), or a YPD culture medium (containing 2% peptone, 1% yeast extract, 0.2% glucose, 1.8% ribose) in which some glucose was substituted with ribose) (hereinafter, referred to as "Culture Method-II" in some cases). Subsequently, using the same method as (1) of Example 1, exocrine secretion of Saccharomyces boulardii in each culture method was recovered.

### (2) Preparation of Mouse Vascular Endothelial Cell Line TKD2 Cell

Subsequently, using the same method as (2) of Example 1, TKD2 cell was sown and cultured overnight.

### (3) Lin28a Expression Induction

Subsequently, TKD2 cell was cultured by using the same method as (3) of Example 1 except that the exocrine secretion (General Culture) of Saccharomyces boulardii, the exocrine secretion (Culture Method I) of Saccharomyces boulardii, or the exocrine secretion (Culture Method II) of Saccharomyces boulardii was added to a culture medium such that a final concentration was 100 µg/mL. Subsequently, each TKD2 cell was recovered, and expression of Lin28a was compared by using the same method as (3) of Example 1. The results are shown in FIG. 2.

From FIG. 2, from General Culture to Culture Method-I and to Culture Method-II, it was apparent that expression of Lin28a was enhanced. In this manner, it was found that by substituting some glucose with rhamnose or ribose, the Lin28a expression inducing effect was significantly enhanced.

### [Example 3]

### (1) Preparation of Culture Product (Exocrine Secretion) of Saccharomyces boulardii

First, exocrine secretion (General Culture) of Saccharomyces boulardii and exocrine secretion (Culture Method-I) of Saccharomyces boulardii were prepared by using the same method as (1) of Example 2.

### (2) Preparation of Human CD14 Positive Monocyte

Subsequently, human CD14 positive monocyte (LONZA Corporation, 2W-400C) was sown in advance in a 10 cm-dish so as to be 1.5 × 10⁶ cells/dish, and cultured overnight at 37°C in a 5% CO2 environment.

### (3) Growth Activation on Human CD14 Positive Monocyte

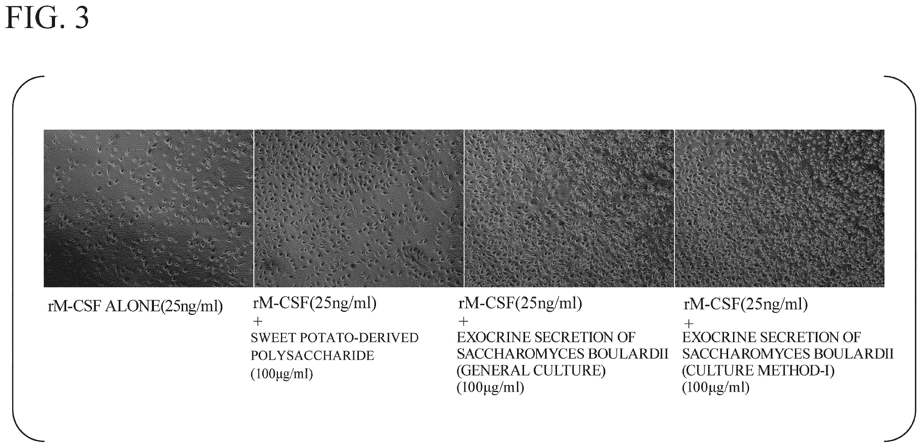

Subsequently, on the day after the date when the human CD14 positive monocyte was sown, a human recombinant M-CSF (rM-CSF) was added to the culture medium such that a final concentration was 25 ng/mL, and the exocrine secretion (Culture Method-I) of Saccharomyces boulardii or the exocrine secretion (Culture Method-II) of Saccharomyces boulardii was added to the culture medium such that the final concentration was 100 µg/mL, and cultured for 2 weeks from the addition. In addition, as a control, a cell added with only rM-CSF was prepared, and as a control group, a group in which a sweet potato-derived polysaccharide was added to the culture medium such that the final concentration was 100 µg/mL was prepared, and cultured for 2 weeks from the addition. FIG. 3 shows aspects of growth of a human mononucleosis after 2 weeks from the addition of various substances to the culture medium.

From FIG. 3, growth activation of monocyte was higher in the cell in which rM-CSF, and the exocrine secretion (General Culture) of Saccharomyces boulardii or the exocrine secretion (Culture Method I) of Saccharomyces boulardii were added to the culture medium than in the cell added with only rM-CSF and the cell in which sweet potato-derived polysaccharide was added to the culture medium such that the final concentration was 100 µg/mL.

In addition, growth activation of monocyte was higher in the cell in which rM-CSF and the exocrine secretion (Culture Method-I) of Saccharomyces boulardii were added to the culture medium than in the cell in which rM-CSF and the exocrine secretion (General Culture) of Saccharomyces boulardii were added to the culture medium.

In this manner, it was found that the effect of activating growth of monocyte becomes higher in proportion to the Lin28a expression inducing effect.

### [Example 4]

Examination was performed on whether there is the same Lin28a activating effect regarding a culture product of microorganisms other than Saccharomyces boulardii. Specifically, spirulina algae, two kinds of Lactobacilluses (Enterococcus saccharolyticus ATCC43076 and Lactobacillales rhamnosus ATCC7469), and Aspergillus (Aspergillus brasiliensis ATCC16404) were used as microalgae.

### (1) Preparation of Culture Product of Microorganisms

### (1-1) Preparation of Culture Product (Exocrine Secretion) of Lactobacillus and Aspergillus

Lactobacillus and Aspergillus were cultured with shake overnight at room temperature using a No. 804 culture medium referring to the website of the independent administrative institution National Institute of Technology and Evaluation (http://www.nite.go.jp/nbrc/cultures/cultures/culture-list.html). The No. 804 culture medium was prepared by dissolving 5 g of high polypeptone, 5 g of yeast extract, 5 g of glucose, and 1 g of MgSO₄·7H₂O in 1 L of water and sterilizing thereof. Subsequently, in the logarithmic growth phase, centrifugation (1,000 g, 15 minutes at room temperature) was performed, and a culture supernatant was recovered. Subsequently, powdered ammonium sulfate was added to the culture supernatant so as to be 75% saturated ammonium sulfate (516 g with respect to 1 L of sample), the resultant product was cooled in ice water for 1 or more hours to recover a precipitate by centrifugation (1,000 g, 15 minutes at 4°C). Subsequently, the recovered precipitate was dissolved in a small amount of distilled water, dialysis was performed against enough water using a dialysis membrane having a limit molecular weight of 3,500, and remaining ammonium sulfate was removed. After the dialysis, exocrine secretions of Lactobacillus and Aspergillus were obtained by freeze-drying.

In addition, as a control, a culture product (exocrine secretion) of Saccharomyces boulardii was also prepared by using the same method as (1) of Example 1.

### (1-2) Preparation of Culture Product or Spirulina Genus Algae

Spirulina genus algae were cultured with shake at room temperature overnight by using a Zarrouk culture medium. Subsequently, in the logarithmic growth phase, centrifugation (1,000 g, 15 minutes at room temperature) was performed, and a bacterial cell was recovered and subjected to freeze-drying. Subsequently, distilled water (25 mL per 1 g of powder) was added to the obtained freeze-dried body (powder), and suspended well. Subsequently, the suspension liquid was subjected to ultrasonication for 10 minutes to prepare a cell crush liquid. Subsequently, the cell crush liquid was added with an equivalent amount of mixed organic solvent (chloroform: methanol = 2:1) of chloroform and methanol, and was shaken for two minute intensely. Subsequently, the resultant product was subjected to centrifugation (2,000 rpm, 15 minutes at room temperature), and separated to obtain a water phase fraction. Subsequently, dialysis was performed on the obtained water phase fraction against enough water using a dialysis membrane having a limit molecular weight of 3,500. After the dialysis, a culture product (extract) of spirulina genus algae was obtained by freeze-drying.

### (2) Preparation of Human Fibroblast

A fibroblast derived from human gingiva was sown in a 6-well plate in advance so as to be 4 × 10⁴ cells/well, and cultured at 37°C overnight in a 5% CO₂ environment by using FBS-containing DMEM (manufactured by Thermo Fisher Scientific Corporation).

### (3) Lin28a Expression Induction

Subsequently, on the day after the date when the human fibroblast was sown, each culture product of microorganisms prepared in (1) was added to a culture medium such that a final concentration was 30 µg/mL, and cultured for 1 to 2 weeks while performing addition. After the addition, the culture medium was not exchanged at all.

After culturing, the cell was isolated and recovered by using a 0.25% Trypsin-EDTA solution (manufactured by Thermo Fisher Corporation, 25200056). Subsequently, from the recovered cell, total RNA was recovered by using RNeasy Micro Kit (QIAGEN 74004). From to total RNA, cDNA was produced by using ImProm-II (registered trademark) reverse Transcription System (Promega A3801). Expression of Lin28a was monitored by PCR using primers shown in the following Table 2. In addition, the PCR condition is as shown below.
1) 95°C for 3 minutes
2) 95°C for 30 seconds
3) 55°C for 30 seconds
4) 72°C for 45 seconds
5) 72°C for 5 minutes
* 30 cycles of 2) to 4)

**[Table 2]**

| | Base Sequence (5' → 3') | SEQ No. |
|---|---|---|
| Forward primer | 5'-aatgcaagtgagggttctgg-3' | 3 |
| Reverse primer | 5'-cttggctccatgaatctggt-3' | 4 |

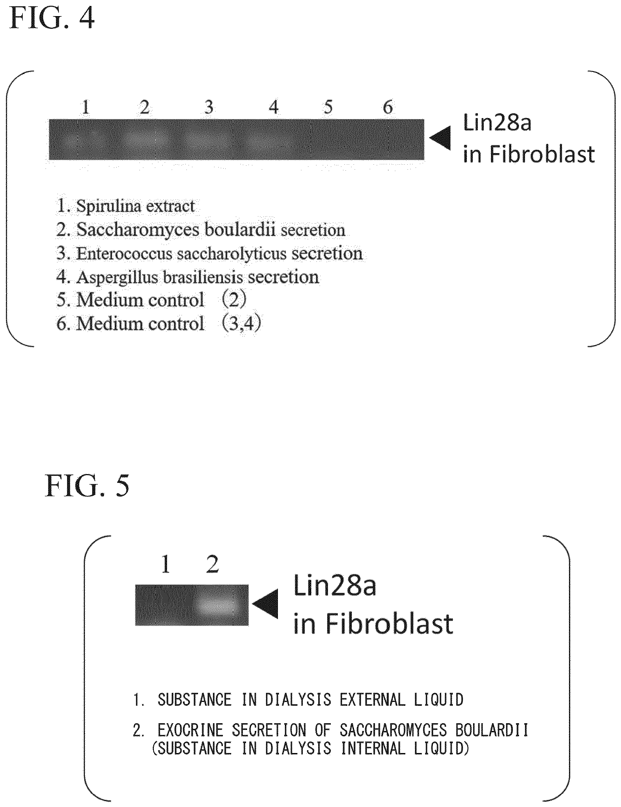

In addition, the reaction liquid after PCR was subjected to agarose gel electrophoresis, and the result was shown in FIG. 4. In FIG. 4, Lane 1 loads fibroblast to which a culture product of spirulina algae was added. Lane 2 loads fibroblast to which exocrine secretion of Saccharomyces boulardii was added. Lane 3 loads fibroblast to which exocrine secretion of Lactobacillus (Enterococcus saccharolyticus ATCC43076) was added. Lane 4 loads fibroblast to which exocrine secretion of Aspergillus (Aspergillus brasiliensis ATCC16404) was added. On the other hand, Lane 5 loads only a YPD culture medium used in culturing of Saccharomyces boulardii as a negative control. Lane 6 also loads only the No. 804 culture medium used in culturing of Lactobacillus and Aspergillus as a negative control.

From FIG. 4, in Lanes 1 to 4 in which fibroblast added with each culture product of microorganisms was loaded, it was checked that expression of Lin28a was induced. In addition, although not shown, in the fibroblast added with Lactobacillus (Lactobacillales rhamnosus ATCC7469) as well, expression of Lin28a was similarly induced.

On the other hand, in Lanes 5 and 6 in which only the culture mediums were loaded, expression of Lin28a was not induced.

### [Example 5]

### (1) Preparation of Culture Product (Exocrine Secretion) of Saccharomyces boulardii

Exocrine secretion (substance in dialysis internal liquid) of Saccharomyces boulardii was obtained by using the same method as (1) of Example 1. In addition, the dialysis external liquid was also recovered, and a substance in the dialysis external liquid was obtained by freeze-drying.

### (2) Preparation of Human Fibroblast

A human fibroblast was cultured in advance by using the same method as (2) of Example 4.

### (3) Lin28a Expression Induction

Lin28a expression inducing activity by each substance was evaluated by using the same method as (3) of Example 4 except that each of exocrine secretion (substance in dialysis internal liquid) of Saccharomyces boulardii obtained in (1) and a substance in the dialysis external liquid was added to the human fibroblast by 30 µg/mL. The results are shown in FIG. 5.

From FIG. 5, in the substance in the dialysis external liquid, Lin28a expression inducing activity was not found. On the other hand, since purification was performed by using a dialysis membrane of which the molecular weight exclusion limit is 3,500 in (1), it was suggested that molecules having a molecular weight of greater than 3,500 contained in the exocrine secretion of Saccharomyces boulardii is an active body.

### [Example 6]

Molecules having Lin28a inducing activity was purified from the culture product of microorganisms, and the composition was analyzed.

### (1) Preparation of Culture Product (Exocrine Secretion) of Saccharomyces boulardii

Exocrine secretion of Saccharomyces boulardii was obtained by the same method as (1) of Example 1.

### (2) Purification Using Anion Exchange Chromatography

Subsequently, the obtained exocrine secretion of Saccharomyces boulardii was fractionated by anion exchange chromatography using DEAE-650M (manufactured by TOSOH BIOSCIENCE CORPORATION, 0007473). Elution was performed by a stepwise method using NaCl of 50 mM, 100 mM, 200 mM, 300 mM, 500 mM, and 2,000 mM. Dialysis was performed on each of the obtained elution fraction against enough water using a dialysis membrane having a limit molecular weight of 3,500. After the dialysis, each elution substance was obtained by freeze-drying. In addition, weight of each of the obtained elution substances was measured.

### (3) Preparation of Human Fibroblast

Human fibroblast was cultured in advance by using the same method as (2) of Example 4.

### (4) Lin28a Expression Induction

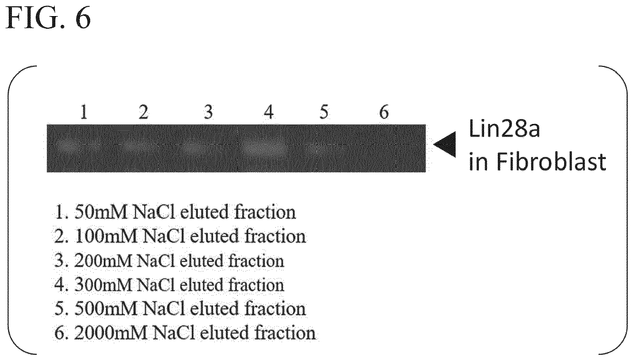

Lin28a expression inducing activity by each elution substance was evaluated by using the same method as (3) of Example 4 except that each elution substance obtained in (1) was added to the human fibroblast by 30 µg/mL. The results are shown in FIG. 6. In FIG. 6, Lanes 1 to 6 load a fibroblast to which the elution substance by NaCl of 50 mM, 100 mM, 200 mM, 300 mM, 500 mM, and 2,000 mM was respectively added.

From FIG. 6, it was apparent that Lin28a expression inducing activity in the fibroblast of the elusion substance by NaCl of 300 mM was the highest.

In addition, the elution substance was dyed with Alcian blue and Toluidine blue, and further tinged with reddish brown with phenol sulfate. From this, it was suggested that the elution substance is a polysaccharide.

### (5) Monosaccharide Composition Analysis

Subsequently, after dialysis of the exocrine secretion (Sample No. 1) of Saccharomyces boulardii obtained in (1), 30 µL of ultrapure water was added to 20 µL of the solution before freeze-drying (1 mg/mL as a sugar-containing amount) to prepare a 50 µL of sample. In addition, 5 µL of the solution (4 mg/mL as a sugar-containing amount) after dialysis by NaCl of 300 mM and before freeze-drying of the elution substance (Sample No. 2) obtained in (2) was added to 45 µL of ultrapure water to prepare a 50 µL of sample. Each of the 50 µL of samples was added with 8M Trifluoroacetic acid, heated at 100°C for 3 hours to hydrolyze. Subsequently, each of obtained hydrolysates was dried and hardened, and dissolved in 100 µL of ultrapure water. The solution was centrifuged (10,000 ×g at 4°C for 10 minutes), and a supernatant was recovered. 50 µL of the obtained supernatant was N-acetylated using acetic anhydride. Subsequently, the resultant product was fluorescently labeled by using an ABEE reagent. Subsequently, water/chloroform extraction was performed on the fluorescently-labeled solution, a fluorescently-labeled monosaccharide was recovered from the aqueous phase, and analysis was performed by high performance liquid chromatography (HPLC). The HPLC condition is as shown below.

Device: BioAssist eZ (manufactured by Tosoh Corporation)
Column: PN-PAK C18 (3.0 × 75 mm)
Solution: boric acid buffer/acetonitrile
Flow rate: 0.5 mL/minute
Detection: fluorescence (Ex: 305 nm, Em: 360 nm)

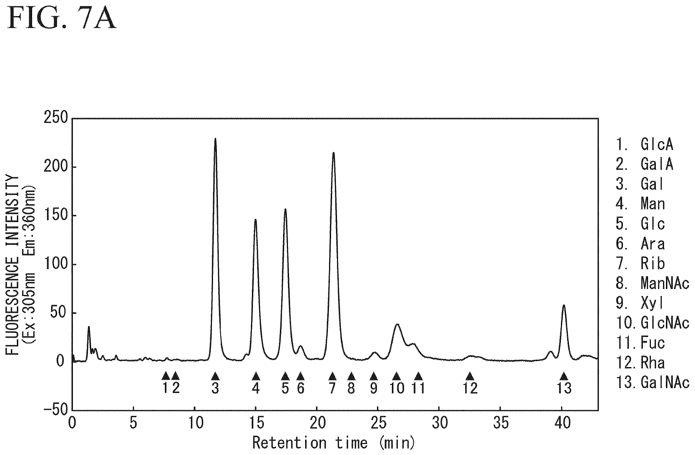

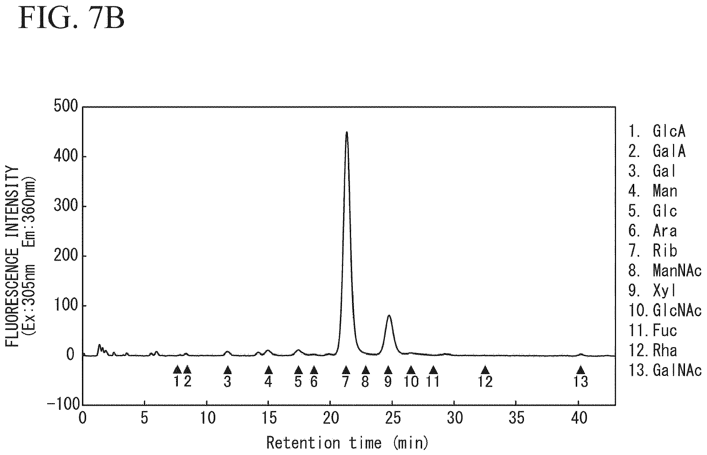

Analysis result of the exocrine secretion (Sample No. 1) of Saccharomyces boulardii obtained in (1) is shown in Table 3 and FIG. 7A. In addition, analysis result of the elution substance (Sample No. 2) by NaCl of 300 mM obtained in (2) is shown in Table 4 and FIG. 7B.

**[Table 3]**

| Sample name: No. 1 | | | | | |
|---|---|---|---|---|---|
| Sign | Ingredient name | Retention time (minute) | pmol | Per 1 mL of sample | |
| | | | | nmol | µg |
| 1 | Glucuronic acid | 7.7 | <5.0 | <67.5 | <13.1 |
| 2 | Galacturonic acid | 8.5 | <20.0 | <270.0 | <52.4 |
| 3 | Galactose | 11.7 | 53.0 | 714.9 | 128.8 |
| 4 | Mannose | 15.0 | 43.4 | 585.5 | 105.5 |
| 5 | Glucose | 17.4 | 40.2 | 543.1 | 97.8 |
| 6 | Arabinose | 18.7 | 4.3 | 58.7 | 8.8 |
| 7 | Ribose | 21.4 | 107.1 | 1445.7 | 217.0 |
| 8 | N-acetyl-mannosamine | ND | ND | ND | ND |
| 9 | Xylose | 24.7 | 3.4 | 45.4 | 6.8 |
| 10 | N-acetyl-glucosamine | 26.6 | 19.2 | 259.7 | 57.4 |
| 11 | Fucose | ND | ND | ND | ND |
| 12 | Rhamnose | 32.6 | 2.7 | 36.1 | 5.9 |
| 13 | N-acetyl-galactosamine | 40.2 | 11.0 | 148.2 | 32.8 |

**[Table 4]**

| Sample name: No. 2 | | | | | |
|---|---|---|---|---|---|
| Sign | Ingredient name | Retention time (minute) | pmol | Per 1 mL of sample | |
| | | | | nmol | µg |
| 1 | Glucuronic acid | ND | ND | ND | ND |
| 2 | Galacturonic acid | 8.3 | <20.0 | <1080.0 | <209.7 |
| 3 | Galactose | 11.6 | 1.7 | 92.7 | 16.7 |
| 4 | Mannose | 15.0 | <1.0 | <54.0 | <9.7 |
| 5 | Glucose | 17.4 | 2.8 | 149.2 | 26.9 |
| 6 | Arabinose | 18.7 | <1.0 | <54.0 | <8.1 |
| 7 | Ribose | 21.4 | 226.4 | 12224.3 | 1835.2 |
| 8 | N-acetyl-mannosamine | ND | ND | ND | ND |
| 9 | Xylose | 24.8 | 34.1 | 1843.2 | 276.7 |
| 10 | N-acetyl-glucosamine | 26.5 | 2.4 | 129.2 | 28.6 |
| 11 | Fucose | ND | ND | ND | ND |
| 12 | Rhamnose | ND | ND | ND | ND |
| 13 | N-acetyl-galactosamine | 40.2 | <1.0 | <54.0 | <11.9 |

From Tables 3 and 4 and FIGS. 7A and 7B, it was apparent that the polysaccharide contained in the elution substance by NaCl of 300 mM having high Lin28a expression inducing activity includes ribose and xylose. In addition, it was apparent that the polysaccharide contained in the exocrine secretion of Saccharomyces boulardii includes galactose, mannose, glucose, N-acetylglucosamine, rhamnose, and N-acetylgalactosamine, in addition to ribose and xylose.

From these, it was apparent that the polysaccharide having Lin28a expression inducing activity at least includes ribose and xylose.

From the above description, it was checked that the polysaccharide contained in the Lin28a activator of the present embodiment has Lin28a expression inducing activity in cells and further has prominent monocyte growth activity.

### [Example 7]

In the human fibroblast cultured by adding the exocrine secretion of Saccharomyces boulardii, gene expression other than Lin28a was analyzed.

### (1) Preparation of Culture Product (Exocrine Secretion) of Saccharomyces boulardii

Exocrine secretion of Saccharomyces boulardii was obtained by using the same method as (1) of Example 1.

### (2) Preparation of Human Fibroblast

Human fibroblast was cultured in advance by using the same method as (2) of Example 4.

### (3) Expression Analysis of Sirt1 Gene and TERT Gene

Subsequently, on the day after the date when the human fibroblast was sown, the exocrine secretion of Saccharomyces boulardii prepared in (1) was added to a culture medium such that a final concentration was 10 µg/mL or 30 µg/mL, and cultured for 1 week from the addition. After the addition, the culture medium was not exchanged at all.

Subsequently, total RNA was recovered from the cell cultured in each condition by using the same method as described in (4) of Example 4 to produce cDNA. Subsequently, expression of Sirt1 gene and TERT gene was monitored by PCR by using the same method as the method described in (4) of Example 4, except that the primer shown in Table 2 and the primer shown in Table 5 were used. The Sirt1 gene is a gene of Sirtuin 1 known as a rejuvenation gene. In addition, the TERT gene is a gene of a telomerase subunit.

**[Table 5]**

| Target gene | | Base sequence (5' → 3') | SEQ No. |
|---|---|---|---|
| Sirtl | Forward primer | 5'-tcagtggctggaacagtgag-3' | 5 |
| | Reverse primer | 5' -tctggcatgtcccactatca-3' | 6 |
| TERT | Forward primer | 5'-agagtgtctggagcaagttgc-3' | 7 |
| | Reverse primer | 5'-cgtagtccatgttcacaatcg-3' | 8 |

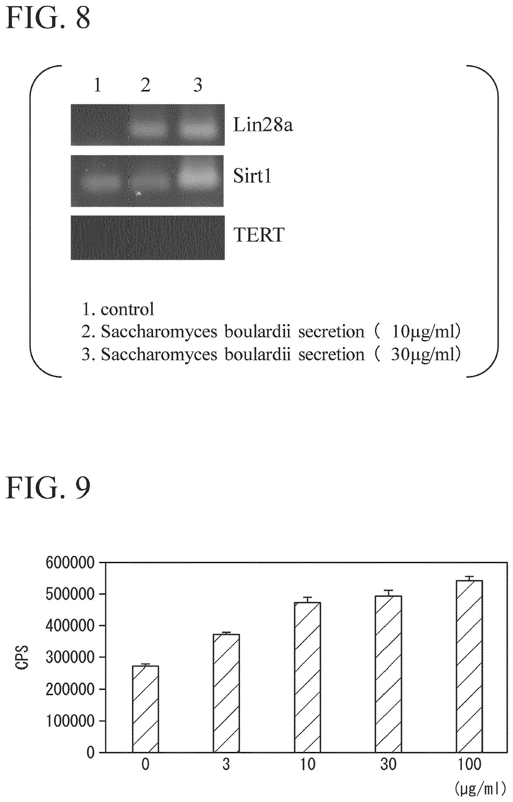

In addition, the reaction liquid after PCR was subjected to agarose gel electrophoresis, and the result was shown in FIG. 8. In FIG. 8, Lane 1 loads fibroblast to which exocrine secretion of Saccharomyces boulardii was added. Lane 2 loads fibroblast to which exocrine secretion (10 µg/mL) of Saccharomyces boulardii was added. Lane 3 loads fibroblast to which exocrine secretion (30 µg/mL) of Saccharomyces boulardii was added.

From FIG. 8, it was apparent that expression induction of Sirt1 gene known as a rejuvenation gene increases depending on an addition amount of the exocrine secretion of Saccharomyces boulardii. From this, it was suggested that the polysaccharide contained in the exocrine secretion of Saccharomyces boulardii can be expected to have a rejuvenating effect.

On the other hand, an effect on the expression of TERT gene in the telomerase subunit was found. From this, it was estimated that the risk of carcinogenesis by the addition of the exocrine secretion of Saccharomyces boulardii was significantly decreased.

### [Example 8]

Although data is not shown, even if the polysaccharide derived from microorganisms was added to a culture liquid, it had no effect on the growth of human fibroblast at all. With respect to this, the effect of the polysaccharide derived from microorganisms on undifferentiated cells was inspected.

### (1) Preparation of Culture Product (Exocrine Secretion) of Saccharomyces boulardii

Exocrine secretion of Saccharomyces boulardii was obtained by using the same method as (1) of Example 1.

### (2) Preparation of Rat Bone Marrow Cell

From a transgenic rat produced such that Luciferase is expressed in the entire body, a bone marrow cell was obtained and cultured. Subsequently, attached cells were recovered from trypsin (manufactured by Thermo Fisher Scientific Corporation, Trypsin-EDTA, 25200056), and culturing was continued. The culturing was performed by using FBS-containing DMEM having a final concentration of 20% (manufactured by Thermo Fisher Scientific Corporation, 21063).

### (3) Culturing Using Culture Product (Exocrine Secretion) of Saccharomyces boulardii

Subsequently, to the bone marrow cell cultured in (2) in advance, the exocrine secretion of Saccharomyces boulardii obtained in (1) was added to a culture medium such that a final concentration was 0, 3, 10, 30, and 100 µg/mL, and cultured for 4 days from the addition. After the addition, the culture medium was not exchanged at all. Subsequently, the number of cells after culturing was quantified. The quantification of the number of cells was performed using fluorescence of luciferin as an index. The result is shown in FIG. 9. In FIG. 9, the axis of ordinates is cps (count per second) of fluorescence of luciferin. The axis of abscissa is an addition concentration of the exocrine secretion of Saccharomyces boulardii.

From FIG. 9, it was apparent that the number of cells of the bone marrow cells increases depending on an addition concentration of the exocrine secretion of Saccharomyces boulardii. From this, it was suggested that by using the above-described polysaccharide derived from microorganisms, when collecting undifferentiated cells such as bone marrow which are noted as a source of cell treatment from a human body, the collection can be performed with minimum invasiveness. In addition, it was suggested that by using the above-described polysaccharide derived from microorganisms, undifferentiated cells effective in treatment is caused to be grown in the human body, that is, it is also effective to realize regeneration treatment not requiring cell culturing outside the body.

### Industrial Applicability

According to the present embodiment, it is possible to provide an effective Lin28a activator and an efficient dedifferentiation inducer.

In addition, the therapeutic agent of the present embodiment includes a polysaccharide containing one or more selected from the group consisting of ribose, xylose, and derivatives thereof, and can activate Lin28a. With this, it is possible to effectively treat diseases relating to damage in cells, tissues, or organs with low invasiveness compared to surgery including transplantation or cell medical care.

In addition, the food or beverage product of the present embodiment includes a polysaccharide containing one or more selected from the group consisting of ribose, xylose, and derivatives thereof, and can activate Lin28a. With this, it is possible to effectively prevent or alleviate diseases relating to damage in cells, tissues, or organs. In addition, from a viewpoint of safety, consuming the above-described polysaccharide has been experienced, and it is considered that it is not problematic to consume the polysaccharide on a daily basis. Therefore, it is possible to provide a safe and effective food or beverage product.

In addition, the cosmetic product of the present embodiment includes a polysaccharide containing one or more selected from the group consisting of ribose, xylose, and derivatives thereof, and can activate Lin28a. With this, the cosmetic product of the present embodiment has excellent anti-inflammatory effect, whitening effect, and anti-aging effect. In addition, since the above-described polysaccharide is harmless, it is not problematic to percutaneously consume the product on a daily basis. Therefore, it is possible to provide a safe and effective cosmetic product.

## Claims

1. A Lin28a activator comprising, as an active ingredient:
a polysaccharide containing one or more selected from the group consisting of ribose, xylose, and derivatives thereof.

2. The Lin28a activator according to Claim 1,
wherein the polysaccharide further contains one or more selected from the group consisting of galactose, mannose, glucose, N-acetylglucosamine, rhamnose, N-acetylgalactosamine, and derivatives thereof.

3. The Lin28a activator according to Claim 1 or 2,
wherein a molecular weight of the polysaccharide is 3,500 or greater.

4. The Lin28a activator according to any one of Claims 1 to 3,
wherein the polysaccharide is derived from microorganisms.

5. The Lin28a activator according to Claim 4,
wherein the microorganism is yeast, microalgae, Lactobacillus, or Aspergillus.

6. The Lin28a activator according to Claim 5,
wherein the yeast is Saccharomyces boulardii.

7. A dedifferentiation inducer of a monocyte comprising, as active ingredients:
a macrophage colony-stimulating factor; and
a polysaccharide containing one or more selected from the group consisting of ribose, xylose, and derivatives thereof.

8. The dedifferentiation inducer of a monocyte according to Claim 7,
wherein the polysaccharide further contains one or more selected from the group consisting of galactose, mannose, glucose, N-acetylglucosamine, rhamnose, N-acetylgalactosamine, and derivatives thereof.

9. The dedifferentiation inducer of a monocyte according to Claim 7 or 8,
wherein a molecular weight of the polysaccharide is 3,500 or greater.

10. A therapeutic agent against diseases relating to damage in cells, tissues, or organs, comprising, as an active ingredient:
a polysaccharide containing one or more selected from the group consisting of ribose, xylose, and derivatives thereof.

11. The therapeutic agent according to Claim 10,
wherein the disease is external injury, pancreatitis, radiation damage, dermatomyositis, polymyositis, necrotizing fasciitis, chronic bronchitis, fracture, osteoporosis, osteochondral fracture, osteochondritis, dilated cardiomyopathy, myocardial infarction, ischemic cardiomyopathy, heart failure, myocardial hypertrophy, congestive heart failure, restenosis, arrhythmia, atherosclerosis, vasculitis, peripheral neuropathy, neuropathic pain, stroke, encephalitis, meningitis, diabetic neuropathy, attention deficit disorder, autism, Alzheimer's disease, Parkinson's disease, Creutzfeldt-Jakob disease, brain trauma, spinal cord trauma, cerebral ischemia, cirrhosis, chronic hepatitis, chronic renal failure, glomerular nephritis, renal ischemia, diabetes, atopic dermatitis, or graft versus host disease.

12. A food or beverage product for preventing or alleviating diseases relating to damage in one of the group consisting of cells, tissues, and organs, comprising:
a polysaccharide containing one or more selected from the group consisting of ribose, xylose, and derivatives thereof.

13. The food or beverage product according to Claim 12,
wherein the disease is external injury, pancreatitis, radiation damage, dermatomyositis, polymyositis, necrotizing fasciitis, chronic bronchitis, fracture, osteoporosis, osteochondral fracture, osteochondritis, dilated cardiomyopathy, myocardial infarction, ischemic cardiomyopathy, heart failure, myocardial hypertrophy, congestive heart failure, restenosis, arrhythmia, atherosclerosis, vasculitis, peripheral neuropathy, neuropathic pain, stroke, encephalitis, meningitis, diabetic neuropathy, attention deficit disorder, autism, Alzheimer's disease, Parkinson's disease, Creutzfeldt-Jakob disease, brain trauma, spinal cord trauma, cerebral ischemia, cirrhosis,
chronic hepatitis, chronic renal failure, glomerular nephritis, renal ischemia, diabetes, atopic dermatitis, or graft versus host disease.

14. A cosmetic product comprising:
a polysaccharide containing one or more selected from the group consisting of ribose, xylose, and derivatives thereof.


SEQUENCE LISTING

<110>  TES HOLDINGS CO., LTD

<120>  LIN28A ACTIVATOR AND USE THEREOF

<130>  PC24219

<160>  6

<170>  PatentIn version 3.5

<210>  1
<211>  20
<212>  DNA
<213>  Artificial Sequence

<220>
<223>  Synthetic DNA

<400>  1
cagaagcgaa gatccaaagg                                                    20

<210>  2
<211>  20
<212>  DNA
<213>  Artificial Sequence

<220>
<223>  Synthetic DNA

<400>  2
caggctttcc ctgagaactg                                                    20

<210>  3
<211>  20
<212>  DNA
<213>  Artificial Sequence

<220>
<223>  Synthetic DNA

<400>  3
tcagtggctg gaacagtgag                                                    20

<210>  4
<211>  20
<212>  DNA
<213>  Artificial Sequence

<220>
<223>  Synthetic DNA

<400>  4
tctggcatgt cccactatca                                                    20

<210>  5
<211>  21
<212>  DNA

<213>  Artificial Sequence

<220>
<223>  Synthetic DNA

<400>  5
agagtgtctg gagcaagttg c                                                 21


<210>  6
<211>  21
<212>  DNA
<213>  Artificial Sequence

<220>
<223>  Synthetic DNA

<400>  6
cgtagtccat gttcacaatc g                                                 21

SEQUENCE LISTING

<110>  TES HOLDINGS CO., LTD

<120>  LIN28A ACTIVATOR AND USE THEREOF

<130>  TESH-001-EP

<150>  PCT/JP2017/033257
<151>  2017-09-14

<160>  6

<170>  PatentIn version 3.5

<210>  1
<211>  20
<212>  DNA
<213>  Artificial Sequence

<220>
<223>  Synthetic DNA

<400>  1
cagaagcgaa gatccaaagg                                                   20


<210>  2
<211>  20
<212>  DNA
<213>  Artificial Sequence

<220>
<223>  Synthetic DNA

<400>  2
caggctttcc ctgagaactg                                                   20


<210>  3
<211>  20
<212>  DNA
<213>  Artificial Sequence

<220>
<223>  Synthetic DNA

<400>  3
tcagtggctg gaacagtgag                                                   20


<210>  4
<211>  20
<212>  DNA
<213>  Artificial Sequence

<220>
<223>  Synthetic DNA

<400>  4
tctggcatgt cccactatca                                                   20

<210>  5
<211>  21
<212>  DNA
<213>  Artificial Sequence

<220>
<223>  Synthetic DNA

<400>  5
agagtgtctg gagcaagttg c                                                21


<210>  6
<211>  21
<212>  DNA
<213>  Artificial Sequence

<220>
<223>  Synthetic DNA

<400>  6
cgtagtccat gttcacaatc g                                                21